(19) Europäisches Patentamt
European Patent Office
Office européen des brevets

(11) **EP 3 394 018 B1**

(12) **FASCICULE DE BREVET EUROPEEN**

(45) Date de publication et mention de la délivrance du brevet:
**21.06.2023 Bulletin 2023/25**

(21) Numéro de dépôt: **16822130.7**

(22) Date de dépôt: **14.12.2016**

(51) Classification Internationale des Brevets (IPC):
*C07C 17/25* (2006.01)    *C07C 17/38* (2006.01)
*C07C 19/08* (2006.01)    *C07C 21/18* (2006.01)
*C09K 5/04* (2006.01)    *C07C 17/386* (2006.01)

(52) Classification Coopérative des Brevets (CPC):
(C-Sets disponibles)
**C07C 17/386;** C09K 5/045; C09K 2205/126   (Cont.)

(86) Numéro de dépôt international:
**PCT/EP2016/080943**

(87) Numéro de publication internationale:
**WO 2017/108517 (29.06.2017 Gazette 2017/26)**

(54) **PROCEDE DE PREPARATION DU 2,3,3,3-TETRAFLUOROPROPENE ET RECYCLAGE DU 1,1,1,2,2-PENTAFLUOROPROPANE EXEMPT D'IMPURETES**

VERFAHREN ZUR HERSTELLUNG VON 2,3,3,3-TETRAFLUORPROPEN UND RÜCKGEWINNUNG VON 1,1,1,2,2-PENTAFLUROPROPAN OHNE VERUNREINIGUNGEN

METHOD FOR PRODUCING 2,3,3,3-TETRAFLUOROPROPENE AND RECYCLING 1,1,1,2,2-PENTAFLUOROPROPANE FREE OF IMPURITIES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.12.2015 FR 1563163**

(43) Date de publication de la demande:
**31.10.2018 Bulletin 2018/44**

(73) Titulaire: **Arkema France**
**92700 Colombes (FR)**

(72) Inventeurs:
• **BABA-AHMED, Abdelatif**
**69190 Saint-fons (FR)**
• **COLLIER, Bertrand**
**69230 Saint-genis-laval (FR)**
• **DEUR-BERT, Dominique**
**69390 Charly (FR)**
• **WENDLINGER, Laurent**
**69510 Soucieu En Jarrest (FR)**

(74) Mandataire: **Arkema Patent**
**Arkema France**
**DRD-DPI**
**420, rue d'Estienne d'Orves**
**92705 Colombes Cedex (FR)**

(56) Documents cités:
**EP-A- 0 921 109        EP-B1- 0 743 934**
**WO-A1-2014/147313   WO-A1-2016/080283**
**WO-A2-2010/123154   US-A- 5 470 442**
**US-B2- 7 371 309**

(52) Classification Coopérative des Brevets (CPC):
(Cont.)

C-Sets
**C07C 17/386, C07C 19/08**

## Description

### Domaine technique de l'invention

L'invention se rapporte à un procédé de préparation du 2,3,3,3-tetrafluoro-1-propène et est définie dans les revendications. Plus particulièrement, l'invention se rapporte à un procédé de préparation de 2,3,3,3-tetrafluoro-1-propène incluant la purification et le recyclage du 1,1,1,2,2-pentafluoropropane également issu de la réaction.

### Arrière-plan technologique de l'invention

Les hydrofluorocarbures (HFC) et en particulier les hydrofluorooléfines (HFOs), telles que le 2,3,3,3-tétrafluoro-1-propène (HFO-1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. Les HFO ont été identifiés comme des alternatives souhaitables au HCFC du fait de leurs faibles valeurs d'ODP (Ozone Depletion Potential ou potentiel d'appauvrissement de la couche d'ozone) et de GWP (Global Warming Potential ou potentiel de réchauffement climatique).

La plupart des procédés de fabrication des hydrofluorooléfines font appel à une réaction de déshydrohalogénation et/ou une réaction de fluoration. Ce type de réaction est effectué en phase gazeuse et génère des impuretés qu'il faut par conséquent éliminer pour obtenir le composé désiré dans un degré de pureté suffisant pour les applications visées.

Par exemple, dans le cadre de la production de 2,3,3,3-tétrafluoro-1-propène (HFO-1234yf), la présence d'impuretés telles que le 1-chloro-3,3,3-trifluoro-1-propene (1233zd), 1,3,3,3-tetrafluoro-1-propene (1234ze) et le 1,1,1,3,3-pentafluoropropane (245fa) sont observées. Ces impuretés sont des isomères des composés principaux visant à être obtenus par le procédé de production du 2,3,3,3-tétrafluoro-1-propène outre ce dernier, i.e. 2-chloro-3,3,3-trifluoro-1-propene (1233xf) et 1,1,1,2,2-pentafluoropropane (245cb). Compte tenu des points d'ébullition respectifs du 1-chloro-3,3,3-trifluoro-1-propene (1233zd), 1,3,3,3-tetrafluoro-1-propene (1234ze) et le 1,1,1,3,3-pentafluoropropane (245fa), ceux-ci peuvent s'accumuler dans le réacteur et ainsi empêcher la formation des produits d'intérêt.

La purification de ce type de mélange réactionnel peut être effectuée par différentes techniques connues de l'art antérieur, telles que par exemple la distillation. Cependant lorsque les composés à purifier ont des points d'ébullition trop proches ou lorsque ceux-ci forment des compositions azéotropiques ou quasi-azéotropiques, la distillation n'est pas un procédé efficace. Des procédés de distillation extractive ont ainsi été décrits.

On connaît par EP 0 864 554 un procédé de purification d'un mélange comprenant 1,1,1,3,3-pentafluoropropane (245fa) et 1-chloro-3,3,3-trifluoro-trans-1-propene (1233zd) par distillation en présence d'un solvant ayant un point d'ébullition supérieur à celui de 1-chloro-3,3,3-trifluoro-trans-1-propene.

On connaît par WO 03/068716 un procédé de récupération de pentafluoroéthane à partir d'un mélange comprenant du pentafluoroéthane et du chloropentafluoroéthane par distillation en présence d'hexafluoropropène.

On connaît aussi par WO 98/19982 un procédé de purification du 1,1-difluoroéthane par distillation extractive. Le procédé consiste à mettre en contact un agent d'extraction avec un mélange de 1,1-difluoroéthane et de chlorure de vinyle. L'agent d'extraction est choisi parmi les hydrocarbures, les alcools, les chlorocarbures ayant un point d'ébullition compris entre 10°C et 120°C. WO 2010/123154 décrit un procédé de purification du 1,1,1,2,2-pentafluoropropane par distillation.

Comme mentionné par WO 98/19982, la sélection de l'agent d'extraction peut s'avérer complexe en fonction des produits à séparer. Il existe donc toujours un besoin pour la mise en oeuvre d'un procédé particulier de purification du 1,1,1,2,2-pentafluoropropane.

### Résumé de l'invention

Dans un procédé de production du 2,3,3,3-tetrafluoro-1-propène, le choix de conditions opérationnelles particulières permet de favoriser la présence de certaines impuretés ou d'isomères. Le 2,3,3,3-tetrafluoro-1-propène peut également être en équilibre avec le 1,1,1,2,2-pentafluoropropane (245cb). Isoler et purifier le 1,1,1,2,2-pentafluoropropane (245cb) obtenu revêt donc un intérêt particulier pour favoriser la production de 2,3,3,3-tetrafluoro-1-propène (1234yf). En outre, la présence d'impuretés telles que 1,3,3,3-tetrafluoro-1-propene (1234ze) peut être observée tout comme celle de 1-chloro-3,3,3-trifluoro-1-propene (1233zd), et 1,1,1,3,3-pentafluoropropane (245fa). Ces impuretés peuvent provenir de réactions secondaires induites par des composés produits intermédiairement pendant la production du 2,3,3,3-tetrafluoro-1-propène, et peuvent présenter des propriétés physiques telles que la séparation de celles-ci avec le 1,1,1,2,2-pentafluoropropane (245cb) peut s'avérer complexe. La présente invention permet, outre la production de 2,3,3,3-tetrafluoro-1-propène, la récupération et le recyclage du 1,1,1,2,2-pentafluoropropane (245cb) produit avec une excellente pureté. Optionnellement, le 1,1,1,2,2-pentafluoropropane (245cb) ainsi récupéré peut faire l'objet d'une réaction de déhydrofluoration en absence ou en présence d'acide fluorhydrique pour former du 2,3,3,3-tetrafluoropropene. Le 1,1,1,2,2-pentafluoropropane (245cb) ainsi récupéré peut faire l'objet d'une réaction de fluoration telle que décrite ci-dessous.

Selon un premier aspect, la présente invention fournit un procédé de purification du 1,1,1,2,2-pentafluoropropane (245cb) à partir d'une première composition comprenant du 1,1,1,2,2-pentafluoropropane et trans-1,3,3,3-tetrafluoro-1-propene (1234ze-E) et optionnellement au moins un des composés choisi parmi le groupe consistant en 1,1-difluoroéthane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf), ledit procédé comprenant les étapes de :

a) mise en contact de ladite première composition avec au moins un agent d'extraction organique pour former une seconde composition ;
b) distillation extractive de ladite seconde composition pour former :

i) une troisième composition comprenant ledit agent d'extraction organique, trans-1,3,3,3-tetrafluoro-1-propene (1234ze-E) et optionnellement ledit au moins un des composés choisi parmi le groupe consistant en 1,1-difluoroéthane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z) et 3,3,3-trifluoropropene (1243zf) ; et
ii) un courant comprenant le 1,1,1,2,2-pentafluoropropane,

a) récupération et séparation de ladite troisième composition pour former un courant comprenant ledit agent d'extraction organique et un courant comprenant trans-1,3,3,3-tetrafluoro-1-propene (1234ze-E), et optionnellement ledit au moins un des composés choisi parmi le groupe consistant en 1,1-difluoroéthane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z) et 3,3,3-trifluoropropene (1243zf), de préférence, l'agent d'extraction organique séparé à l'étape c) est recyclé à l'étape a) ; et caractérisé en ce que ledit agent d'extraction organique est sélectionné parmi le groupe consistant en éthylamine, bromofluoromethane, 1-bromo-1,2-difluoroethylene, acetaldehyde, 1,1,1-trifluoro-2-propanone, 1,1,1-trifluoro-2-bromoethane, 2,2,2-trifluoroethylmethylether, isopropylamine, methylformate, 2-methoxy-1-propene, diethylether, 1,2-epoxypropane, ethanethiol, ethoxy-ethene, ethylmethylamine, dimethylsulfide, 2-chloropropane, bromoethane, dimethoxymethane, iodomethane, 2-amino-2-methylpropane, methylcyclopropylether, n-propylamine, isopropylmethylamine, ethanedial, 2-chloro-2-methylpropane, 2-propanethiol, 2-ethoxy-propane, methyl-t-butylether, diethylamine, propanone, methylacetate, 4-methoxy-2-methyl-2-butanethiol, 2-bromopropane,chloromethoxymethane, 2-butanamine, n-methylpropylamine, tert-butylthiol, isobutanal, methanol, tetrahydrofurane, 1-propanethiol, chlorobromomethane, isopropylformate, diisopropylether, 3-bromopropene, 1-bromopropane, methylglyoxal, iodoethane, 2-ethoxy-2-methyl-propane, 2-bromo-2-methylpropane, 2,2,2-trifluoroethanol, 1-chloro-3-fluoropropane, 1,1,1-trifluoro-2-propanol, 1-butylamine, ethylacetate, ethanol, butanone, n-propylformate, 2-ethoxy-butane, 2-propanol, acetonitrile, tert-butanol, 1-methoxy-2-methyl-butane, 2,2-dimethoxypropane, 2-chloro-2-methylbutane, 1,2-dichloroethane, 1-ethoxy-2-methylpropane, diisopropylamine, 2-butanethiol, 1,2-dimethoxyethane, 3-methyl-2-butanamine, diethoxymethane, 2-methyl-1-propanethiol, isopropylacetate, 2-iodopropane, di-n-propylether, 3-pentylamine, n-methylbutylamine, 2-bromobutane, diethylsulfide, 1-ethoxybutane, 1-methoxy-2-propanamine, 2-methylbutanal, 2-methoxyethanamine, 2,2-difluoroethanol, 1,2-dichloropropane, propanol, tert-butylacetate, propionitrile, trichloroacetaldehyde, 2-allyloxyethanol, butanethiol, 1-methoxy-pentane, ethylpropionate, 2-butanol, 1,2-dimethoxypropane, isopropyl-isobutyl-ether, 1-chloro-4-fluorobutane, 1-bromo-3-fluoropropane, dioxane, 1-bromobutane, 3-pentanone, 1,1-diethoxyethane, 2-pentanone, 2-methyl-2-butanol, 1-iodopropane, 2-methoxy-1propanamine, trimethoxymethane, cis-1,3-dichloropropene, n-pentylamine, 3,3-dimethyl-2-butanone, 1,3-dioxane, piperidine, 1-bromo-2-chloroethane, isobutanol, 2-bromo-2-methylbutane, dipropylamine, 2,2,3,3-tetraflouro-1-propanol, 2-ethoxyethanamine, triethylfluorosilane, sec-butylacetate, trans-1,3-dichloropropene, 2,2-dimethyl-1-propanol, n-methyl-1,2-ethanediamine, 2,2-diethoxypropane, 1,3,5-trioxane, pyridine, n-methylmorpholine, 3-pentanol, 4-methyl-2-pentanone, 1,2-diaminoethane, isobutyl-tert-butylether, 2-bromopentane, butyronitrile, 1-butanol, 2,3-dichlorobutane, sec-butyl-tert-butylether, 1-methoxy2-propanol, 1,2-propanediamine, 2,6-dimethyl-5-heptenal, 1-bromo-3-methylbutane, 1,3-dichloro-trans-2-butene, 1,3-dichloropropane, 1-(dimethylamino)-2-propanol, tetrahydrothiophene, 3-methyl-3-pentanol, 1,2-dibromo-1-fluoroethane, 1,1-diethoxypropane, 1,2,2-trichloropropane, 1-chloro-2-methyl-2-propanol, 2-methoxyethanol, 4,4,4-trifluorobutanol, 2-ethylbutylamine, diethylcarbonate, n-butylacetate, 1-pentanethiol, 2-chloro-1,1-dimethoxyethane, 2-hexanone, n-ethylethylenediamine, 3-fluoropropanol, 5-hexen-2-one, 2,3-dichloro-2-methylbutane, 1,1-diethoxy-n,n-dimethylmethanamine, 2-methylpyridine, 1-bromopentane, 2-methoxy1-propanol, 1,2-dichloro-2-butene, 1-iodobutane, hexanal, 1-ethoxy-2-propanol, 1,2-dibromoethane, 4-methyl-2-pentanol, bromoaceticacidmethylester, 1,1,2-trichloropropane, 1,2-octanediol, 4-methyl-2-hexanamine, hexylamine, 2-chloro-1-propanol, methoxycyclohexane, 2-(dimethylamino)-ethanol, 1,3-dichlorobutane, cyclohexylamine, n-ethyl-2-dimethylaminoethylamine, ethoxyethanol, 3-hexanol, 2-hexanol, 2-methylpyrazine, 2-ethoxy-1-propanol, 1-pentanol, n-ethyl-morpholine, 1-methylpiperazine, 1,3-propanediamine, di-n-butylether, valeronitrile, (methyleneamino)acetonitrile, 1,2-dibromopropane, 1,2,3-trichloropropene, 2-heptanamine, 2,3-dimethylbutanol, 1-ethoxy-hexane, 1-chloro-3-bromopropane, n,n-diethylethylenediamine, 3-furfural, 2,6-dimethylpyridine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, 2-ethyl-1-butanol, 2-methyl-1-pentanol, methylhexanoate, 2-propoxyethanol, 1-propoxy-2-propanol, dimethylethanolamine.

Selon un mode de réalisation préféré, la première composition est une composition azéotropique ou quasi-azéotropique comprenant 1,1,1,2,2-pentafluoropropane (245cb) et trans-1,3,3,3-tetrafluoro-1-propene (1234ze-E) et obtenue à une température de -30°C à 50°C à une pression de 0,5 à 10 bars.

Selon un mode de réalisation préféré, ledit agent d'extraction organique est choisi parmi le groupe consistant en éthylamine, isopropylamine, n-propylamine, diethylamine, tetrahydrofurane, ethylacetate, butanone, 3-pentylamine, 2-methoxyethanamine, dioxane, 2-pentanone, n-pentylamine, 1,3-dioxane, 1,2-diaminoethane, 1,2-propanediamine, 2-methoxyethanol, n-butylacetate, 1-ethoxy-2-propanol.

Selon un mode de réalisation préféré, le 1,1,1,2,2-pentafluoropropane séparé à l'étape b) est récupéré pour être utilisé dans un procédé de production du 2,3,3,3-tetrafluoropropène.

Selon un mode de réalisation préféré, ladite première composition mise en oeuvre à l'étape a) est obtenue à partir d'une composition comprenant du 2,3,3,3-tetrafluoro-1-propene (1234yf).

Selon un mode de réalisation préféré, le procédé comprend préalablement à l'étape a) les étapes :

i') mise en oeuvre d'une composition A comprenant 2,3,3,3-tetrafluoro-1-propène, 1,1,1,2,2-pentafluoropropane, et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf), et optionnellement ou non des impuretés ayant un point d'ébullition inférieur au point d'ébullition du 2,3,3,3-tetrafluoro-1-propène ;

ii') optionnellement ou non, distillation de ladite composition de 2,3,3,3-tetrafluoro-1-propène pour éliminer en tête de colonne de distillation des impuretés ayant un point d'ébullition inférieur au point d'ébullition du 2,3,3,3-tetrafluoro-1-propène et former un premier courant comprenant 2,3,3,3-tetrafluoro-1-propène, 1,1,1,2,2-pentafluoropropane, et au moins un des composés choisi parmi le groupe consistant en 1,1-difluoroéthane (152a), 1,1,1,2-tetrafluoro-roethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) récupéré en bas de colonne de distillation ;

iii') distillation dudit premier courant récupéré en bas de colonne de distillation à l'étape ii') ou de la composition A pour former et récupérer en tête de colonne de distillation un second courant comprenant du 2,3,3,3-tetrafluoro-1-propène et pour former et récupérer en bas de colonne de distillation un troisième courant comprenant 1,1,1,2,2-pentafluoropropane, et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) correspondant à ladite première composition mise en oeuvre à l'étape a) du procédé de purification du 1,1,1,2,2-pentafluoropropane selon la présente invention.

Selon un mode de réalisation particulier, ledit troisième courant formé et récupéré à l'étape iii') peut contenir du 2,3,3,3-tétrafluoro-1-propène issu de la composition A ou dudit premier courant obtenu à l'étape ii'), avantageusement en faible proportion exprimée en pourcentage en poids sur base du poids total dudit troisième courant, de préférence dans une proportion inférieure à celle du 1,1,1,2,2-pentafluoropropane exprimée en pourcentage en poids sur base du poids total dudit troisième courant. Avantageusement, ledit troisième courant formé et récupéré à l'étape iii') peut contenir moins de 10% en poids, avantageusement moins de 5% en poids, de préférence moins de 1% en poids de 2,3,3,3-tétrafluoro-1-propène sur base du poids total dudit troisième courant. Dans ce cas, lors de la mise en oeuvre de l'étape a) et b) du présent procédé de purification, le 2,3,3,3-tétrafluoro-1-propène sera récupéré dans le courant formé et récupéré à l'étape b) comprenant le 1,1,1,2,2-pentafluoropropane.

Selon un second aspect de la présente invention, un procédé de production de 2,3,3,3-tetrafluoro-1-propène est fourni. Ledit procédé comprend les étapes de :

A) fluoration en présence d'un catalyseur d'un composé de formule $CX(Y)_2-CX(Y)_m-CH_mXY$ (I) dans laquelle X et Y représentent indépendamment H, F, ou Cl et m = 0 ou 1 ; et/ou fluoration en présence d'un catalyseur d'un composé de formule $(CX_nY_{3-n})CH_pX_{1-p}CH_mX_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant comprenant du 2,3,3,3-tetrafluoro-1-propene, du 1,1,1,2,2-pentafluoropropane, et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) ;

C) distillation du courant récupéré à l'étape B) et récupération en tête de colonne de distillation d'un courant comprenant du 2,3,3,3-tetrafluoro-1-propene et en bas de colonne de distillation un courant comprenant du 1,1,1,2,2-pentafluoropropane et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf);

D) mise en oeuvre du procédé de purification du 1,1,1,2,2-pentafluoropropane selon la présente invention à partir du courant récupéré en bas de colonne de distillation à l'étape C); et

E) recyclage à l'étape A) du courant comprenant du 1,1,1,2,2-pentafluoropropane formé à l'étape b) du procédé de purification 1,1,1,2,2-pentafluoropropane mis en oeuvre à l'étape D) ou déhydrofluoration du courant comprenant du 1,1,1,2,2-pentafluoropropane formé à l'étape b) du procédé de purification 1,1,1,2,2-pentafluoropropane mis en oeuvre à l'étape D) en l'absence ou présence d'acide fluorhydrique.

Selon un autre aspect, l'invention fournit une composition comprenant du 1,1,1,2,2-pentafluoropropane, trans-1,3,3,3-tetrafluoro-1-propene et un agent d'extraction organique sélectionné parmi le groupe consistant en éthylamine, isopropylamine, n-propylamine, diéthylamine, tetrahydrofurane, ethylacetate, butanone, 3-pentylamine, 2-methoxyethanamine, dioxane, 2-pentanone, n-pentylamine, 1,3-dioxane, 1,2-diaminoethane, 1,2-propanediamine, 2-methoxyethanol, n-butylacetate, 1-ethoxy-2-propanol.

Selon un autre aspect, une composition azéotropique ou quasi azéotropique comprenant 50 à 99,99% en poids de 1,1,1,2,2-pentafluoropropane sur base du poids total de la composition et 0,01 à 50 % en poids de trans-1,3,3,3-tetrafluoro-1-propène sur base du poids total de la composition est décrite.

Selon un autre aspect, une composition azéotropique ou quasi azéotropique comprenant 50 à 99,99% en poids de 1,1,1,2,2-pentafluoropropane sur base du poids total de la composition, et 0,01 à 50 % en poids d'au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) sur base du poids total de la composition est décrite.

Selon un autre aspect, une composition azéotropique ou quasi-azéotropique comprenant 50 à 99,99% en poids de 1,1,1,2,2-pentafluoropropane, moins de 50 % en poids de trans-1,3,3,3-tetrafluoro-1-propène sur base du poids total de la composition et au moins un des composés sélectionnés parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) est décrite.

**Brève description des dessins**

Les figures 1a-c représentent schématiquement un dispositif mettant en oeuvre un procédé de purification du 1,1,1,2,2-pentafluoropropane selon un mode de réalisation particulier de la présente invention.

La figure 2 représente schématiquement un dispositif mettant en oeuvre un procédé de production du 2,3,3,3-tetrafluoropropene selon un mode de réalisation particulier de la présente invention.

**Description détaillée de l'invention**

Le terme « hydrocarbure » tel qu'utilisé ici se réfère à des composés linéaires ou branchés d'alcane en $C_1$-$C_{20}$, cycloalcane en $C_3$-$C_{20}$, alcène en $C_5$-$C_{20}$, cycloalcène en $C_5$-$C_{20}$, arène en $C_6$-$C_{18}$. Par exemple, le terme alcane se réfère à des composés de formule $C_nH_{2n+2}$ dans lequel n est compris entre 1 et 20. Le terme alcane en $C_1$-$C_{20}$ englobe par exemple le pentane, hexane, heptane, octane, nonane, décane ou des isomères de ceux-ci. Le terme alcène en $C_5$-$C_{20}$ se réfère à des composés hydrocarbonés comprenant une ou plusieurs doubles liaisons carbone-carbone et comprenant de 5 à 20 atomes de carbone. Le terme cycloalcane en $C_3$-$C_{20}$ se réfère à un cycle hydrocarboné saturé comprenant de 3 à 20 atomes de carbone. Le terme aryle en $C_6$-$C_{18}$ se réfère à des composés hydrocarbonés cycliques et aromatiques comprenant de 6 à 18 atomes de carbone. Le terme cycloalcène en $C_5$-$C_{20}$ se réfère à des composés hydrocarbonés cycliques comprenant de 5 à 20 atomes de carbone et comprenant une ou plusieurs doubles liaisons carbone-carbone.

Le terme « alkyle » désigne un radical monovalent issu d'un alcane, linéaire ou branché, comprenant de 1 à 20 atomes de carbone. Le terme « cycloalkyle » désigne un radical monovalent issu d'un cycloalcane comprenant de 3 à 20 atomes de carbone. Le terme « aryle » désigne un radical monovalent issu d'un arène comprenant de 6 à 18 atomes de carbone. Le terme « alkényle » désigne un radical monovalent de 2 à 20 atomes de carbone et au moins une double liaison carbone-carbone. Le terme « alkynyle » désigne un radical monovalent de 2 à 20 atomes de carbone et au moins une triple liaison carbone-carbone. Le terme « halogène » se réfère à un groupement -F, -Cl, -Br ou -I. Le terme « cycloalkényle » se réfère à un radical monovalent issu d'un cycloalcène comprenant de 3 à 20 atomes de carbone. Les substituants alkyle en $C_1$-$C_{20}$, alkényle en $C_2$-$C_{20}$, alkynyle en $C_2$-$C_{20}$, cycloalkyle en $C_3$-$C_{20}$, cycloalkényle en $C_3$-$C_{20}$, aryle en $C_6$-$C_{18}$ peuvent être substitués ou non par un ou plusieurs substituants -OH, halogène, -NR$^a$C(O)R$^b$, -C(O)NR$^a$R$^b$ -CN, -NO$_2$, -NR$^a$R$^b$, -OR$^a$, -SR$^a$, -CO$_2$R$^a$, -OC(O)OR$^a$, -OC(O)R$^a$, - C(O)H, -C(O)R$^a$, dans lequel R$^a$ et R$^b$ sont indépendamment l'un de l'autre hydrogène, alkyle en $C_1$-$C_{20}$ non substitué, alkényle en $C_2$-$C_{20}$ non substitué, alkynyle en $C_2$-$C_{20}$ non substitué, cycloalkyle en $C_3$-$C_{20}$ non substitué, cycloalkényle en $C_3$-$C_{20}$ non substitué, aryle en $C_6$-$C_{18}$ non substitué. Dans les substituants -NR$^a$R$^b$, R$^a$ et R$^b$ peuvent former avec l'atome d'azote auquel ils sont rattachés un hétérocycle saturé ou insaturé, aromatique ou non, comprenant de 5 à 10 chainons.

Le terme « hydrohalocarbures » se réfère à des composés de formule R$^a$X dans laquelle R$^a$ est sélectionné parmi alkyle en $C_1$-$C_{20}$, alkényle en $C_2$-$C_{20}$, alkynyle en $C_2$-$C_{20}$, cycloalkyle en $C_3$-$C_{20}$, cycloalkényle en $C_3$-$C_{20}$, aryle en $C_6$-$C_{18}$ et X représente un atome de chlore, de fluor, de brome ou d'iode. Les substituants alkyle en $C_1$-$C_{20}$, alkényle en $C_2$-$C_{20}$, alkynyle en $C_2$-$C_{20}$, cycloalkyle en $C_3$-$C_{20}$, cycloalkényle en $C_3$-$C_{20}$, aryle en $C_6$-$C_{18}$ peuvent être substitués ou non par un ou plusieurs substituants -OH, halogène, -NR$^a$C(O)R$^b$, -C(O)NR$^a$R$^b$ -CN, -NO$_2$, -NR$^a$R$^b$, -OR$^a$, - SR$^a$, -CO$_2$R$^a$, -OC(O)OR$^a$, -OC(O)R$^a$, -C(O)H, -C(O)R$^a$, dans lequel R$^a$ et R$^b$ sont tels que définis ci-dessus.

Le terme « alcool » se réfère à des hydrocarbures ou hydrohalocarbures tels que définis ci-dessus dans lequel au moins un atome d'hydrogène est remplacé par un groupement hydroxyle -OH.

Le terme « cétone » se réfère à des hydrocarbures comprenant au moins un ou plusieurs groupements fonctionnels carbonyle R$^c$-C(O)-R$^d$ dans lequel R$^c$ et R$^d$ sont indépendamment l'un de l'autre un alkyle en $C_1$-$C_{20}$, alkényle en $C_2$-$C_{20}$, alkynyle en $C_2$-$C_{20}$, cycloalkyle en $C_3$-$C_{20}$, cycloalkényle en $C_3$-$C_{20}$, aryle en $C_6$-$C_{18}$ peuvent être substitués ou non par un ou plusieurs substituants -OH, halogène, -NR$^a$C(O)R$^b$, -C(O)NR$^a$R$^b$ -CN, -NO$_2$, -NR$^a$R$^b$, -OR$^a$, -SR$^a$, -CO$_2$R$^a$, - OC(O)OR$^a$, -OC(O)R$^a$, -C(O)H, -C(O)R$^a$, dans lequel R$^a$ et R$^b$ sont tels que définis ci-dessus, R$^c$ et R$^d$ pouvant être liés entre eux pour former avec le groupement carbonyle auquel ils sont rattachés une cétone cyclique comprenant de 4 à 10 chainons, de préférence de 4 à 7 chainons. La cétone cyclique peut également comprendre une ou plusieurs doubles liaisons carbone-carbone. La cétone cyclique peut également être substituée ou non par un ou plusieurs substituants tels que définis ci-dessus.

Le terme « amine » se réfère à des hydrocarbures comprenant au moins un ou plusieurs groupements fonctionnels amine -NR$^c$R$^d$ dans lequel R$^c$ et R$^d$ sont tels que définis ci-dessus, R$^c$ et R$^d$ pouvant être liés entre eux pour former avec l'atome d'azote auquel ils sont rattachés un hétérocycle aromatique ou non comprenant de 4 à 10 chainons.

Le terme « esters » se réfère à des composés de formule R$^c$-C(O)-O-R$^d$ dans lequel R$^c$ et R$^d$ sont tels que définis ci-dessus, R$^c$ et R$^d$ pouvant être liés entre eux pour former avec le groupement ester un cycle comprenant de 4 à 20 atomes de carbone.

Le terme « éther » se réfère à des composés de formule R$^c$-O-R$^d$ dans lequel R$^c$ et R$^d$ sont tels que définis ci-dessus, R$^c$ et R$^d$ pouvant être liés entre eux pour former avec l'atome d'oxygène auquel ils sont rattachés un hétérocycle comprenant de 4 à 20 atomes de carbone.

Le terme « aldéhyde » se réfère à des composés comprenant au moins un ou plusieurs groupements fonctionnels -C(O)-H.

Le terme « nitrile » se réfère à des composés comprenant au moins un ou plusieurs groupements fonctionnels -CN.

Le terme « carbonate » se réfère à des composés de formule R$^c$-O-C(O)-O-R$^d$ dans lequel R$^c$ et R$^d$ sont tels que définis ci-dessus.

Le terme « thioalkyle » concerne des composés de formule R$^c$SR$^d$ dans laquelle R$^c$ et R$^d$ sont tels que définis ci-dessus.

Le terme « amide » concerne des composés de formule R$^c$C(O)NR$^e$R$^d$ dans laquelle R$^c$ et R$^d$ sont tels que définis ci-dessus, R$^e$ ayant la même définition que R$^c$, R$^c$ et R$^d$ pouvant être liés entre eux pour former avec le groupement amide -C(O)N- auquel ils sont rattachés une amide cyclique comprenant de 4 à 10 chainons, de préférence de 4 à 7 chainons. L'amide cyclique peut également comprendre une ou plusieurs doubles liaisons carbone-carbone. L'amide cyclique peut également être substituée ou non par un ou plusieurs substituants tels que définis ci-dessus.

Le terme « hétérocycle » désigne un cycle carboné comprenant de 4 à 10 chainons dont au moins un des chainons est un hétéroatome sélectionné parmi le groupe consistant en O, S, P et N. L'hétérocycle peut comprendre un ou plusieurs double liaison carbone-carbone ou une ou plusieurs double liaison carbone-hétéroatome ou une ou plusieurs double liaison hétéroatome-hétéroatome. De préférence, l'hétérocycle peut comprendre 1, 2, 3, 4 ou 5 hétéroatomes tel que défini ci-dessus. En particulier, l'hétérocycle peut comprendre 1, 2 ou 3 hétéroatomes sélectionné parmi l'oxygène, l'azote ou le soufre. De préférence, l'hétérocycle peut être un cycle carboné comprenant de 4 à 6 chaînons dont 1, 2 ou 3 chaînons sont des hétéroatomes sélectionnés parmi O ou N. L'hétérocycle peut être ou non substitué par un ou plusieurs substituants choisi parmi -OH, halogène, $-NR^aC(O)R^b$, $-C(O)NR^aR^b$ -CN, $-NO_2$, $-NR^aR^b$, $-OR^a$, $-SR^a$, $-CO_2R^a$, $-OC(O)OR^a$, $-OC(O)R^a$, -C(O)H, $-C(O)R^a$ dans lequel $R^a$ et $R^b$ sont tels que définis ci-dessus.

Le terme « composition azéotropique » désigne un mélange liquide de deux ou plusieurs composés se comportant comme une substance unique, et qui bout à température fixe en gardant une composition en phase liquide identique à celle de la phase gaz. Le terme « composition quasi-azéotropique » désigne un mélange liquide de deux ou plusieurs composés ayant un point d'ébullition constant ou qui a tendance à ne pas se fractionner lorsqu'il est soumis à l'ébullition ou l'évaporation.

Le terme « agent d'extraction organique » se réfère à un composé comprenant au moins un atome de carbone.

Selon un premier aspect, l'invention se rapporte à un procédé de purification du 1,1,1,2,2-pentafluoropropane (245cb) tel que défini dans les revendications.

Le procédé de purification est effectué à partir d'une première composition comprenant du 1,1,1,2,2-pentafluoropropane et trans-1,3,3,3-tetrafluoro-1-propene (1234ze-E) et optionnellement au moins un des composés choisi parmi le groupe consistant en 1,1-difluoroéthane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf).

De préférence, ledit procédé comprend les étapes de :

a) Mise en contact de ladite première composition avec au moins un agent d'extraction organique pour former une seconde composition ;

b) Distillation extractive de ladite seconde composition pour former :

i) une troisième composition comprenant ledit agent d'extraction organique, trans-1,3,3,3-tetrafluoro-1-propene (1234ze-E) et optionnellement ledit au moins un des composés choisi parmi le groupe consistant en 1,1-difluoroéthane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z) et 3,3,3-trifluoropropene (1243zf) ; et

ii) un courant comprenant le 1,1,1,2,2-pentafluoropropane,

c) récupération et séparation de ladite troisième composition pour former un courant comprenant ledit agent d'extraction organique et un courant comprenant ledit au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z) et 3,3,3-trifluoropropene (1243zf).

L'agent d'extraction séparé à l'étape c) peut être récupéré et recyclé à l'étape a).

Ladite première composition est définie dans les revendications et peut comprendre du 1,1,1,2,2-pentafluoropropane et au moins deux, au moins trois, au moins quatre, au moins cinq, au moins six ou l'ensemble des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf).

Ladite première composition peut comprendre entre 50 et 99,99 % en poids de 1,1,1,2,2-pentafluoropropane sur base du poids total de la première composition, avantageusement entre 60 et 99,9% en poids, de préférence entre 70 et 99,8% et en particulier entre 75 et 99,5% en poids de 1,1,1,2,2-pentafluoropropane sur base du poids total de la première composition.

Lorsqu'elle en contient, ladite première composition peut comprendre moins de 2000 ppm poids de 1,1-difluoroéthane (152a) sur base du poids total de la première composition, avantageusement entre 1 et 1000 ppm poids, de préférence entre 5 et 500 ppm et en particulier entre 10 et 250 ppm en poids de 1,1-difluoroéthane (152a) sur base du poids total de la première composition.

Lorsqu'elle en contient, ladite première composition peut comprendre moins de 2000 ppm poids de 1,1,1,2-tetrafluoroethane (134a) sur base du poids total de la première composition, avantageusement entre 1 et 1000 ppm poids, de préférence entre 5 et 500 ppm et en particulier entre 10 et 250 ppm poids de 1,1,1,2-tetrafluoroethane (134a) sur base du poids total de la première composition.

Ladite première composition peut comprendre moins de 50 % en poids de trans-1,3,3,3-tetrafluoro-1-propene (1234ze-E) sur base du poids total de la première composition, avantageusement entre 0.1 et 40% en poids, de préférence entre 0.2 et 30% et en particulier entre 0.5 et 25% en poids de trans-1,3,3,3-tetrafluoro-1-propene (1234ze-E) sur base du poids total de la première composition.

Lorsqu'elle en contient, ladite première composition peut comprendre moins de 5000 ppm poids de cis-1,3,3,3-tetrafluoro-1-propene (1234ze-Z) sur base du poids total de la première composition, avantageusement entre 1 et 2000 ppm poids, de préférence entre 5 et 1000 ppm et en particulier entre 10 et 500 ppm poids de cis-1,3,3,3-tetrafluoro-1-propene (1234ze-Z) sur base du poids total de la première composition.

Lorsqu'elle en contient, ladite première composition peut comprendre moins de 2000 ppm poids de trans-1,2,3,3,3-pentafluoropropene (1225ye-E) sur base du poids total de la première composition, avantageusement entre 1 et 1000 ppm poids, de préférence entre 5 et 500 ppm et en particulier entre 10 et 250 ppm poids de trans-1,2,3,3,3-pentafluoropropene (1225ye-E) sur base du poids total de la première composition.

Lorsqu'elle en contient, ladite première composition peut comprendre moins de 2000 ppm poids de cis-1,2,3,3,3-pentafluoropropene (1225ye-Z) sur base du poids total de la première composition, avantageusement entre 1 et 1000 ppm poids, de préférence entre 5 et 500 ppm et en particulier entre 10 et 250 ppm poids de cis-1,2,3,3,3-pentafluoropropene (1225ye-Z) sur base du poids total de la première composition.

Lorsqu'elle en contient, ladite première composition peut comprendre moins de 1 % en poids de 3,3,3-trifluoropropene (1243zf) sur base du poids total de la première composition, avantageusement entre 1 et 5000 ppm poids, de préférence entre 5 et 2000 ppm et en particulier entre 10 et 1500 ppm poids de 3,3,3-trifluoropropene (1243zf) sur base du poids total de la première composition.

L'invention est définie dans les revendications. Également décrit est un agent d'extraction organique qui est un solvant choisi parmi le groupe consistant en hydrohalocarbure, alcool, cétone, amine, ester, éther, aldéhyde, nitrile, carbonate, thioalkyle, amide et hétérocycle. En particulier, ledit agent d'extraction organique peut être un solvant organique inerte choisi parmi le groupe consistant en alcool, cétone, amine, ester et hétérocycle. De préférence, l'hétérocycle est un hétérocycle comprend de 4 à 6 chaînons dont 1, 2 ou 3 chaînons sont des hétéroatomes tels que O ou N.

De préférence, les hydrohalocarbures sont sélectionnés parmi le groupe consistant en Iodomethane, bro-moethane, chlorobromomethane, iodoethane, 2-bromopropane, dichlorobromomethane, 2-chloropropane, 2-iodopropane, bromotrichloromethane, trichloroacetaldehyde, 1,2-dibromopropane, 2-bromobutane, 1,2-dichloropropane, 1,1,2-trichloroethane, 1,2,3-trichloropropene, 1,2-dibromoethane, 1-bromopropane, 3-bromopropene, 1-bromo-2-chloroethane, 1,2-dichloroethane, 1-iodopropane, 2-bromopentane, 1-bromo-3-methylbutane, tribromomethane, 1-bromobutane, 1-chloro-3-bromopropane, 1-bromopentane, 1,3-dichloropropane, 1-bromo-3-fluoropropane, 1,2-dibromo-1-fluoroethane, 1-bromo-1,2-difluoroethylene, bromofluoromethane, 1,1,1-trifluoro-2-bromoethane, 1-chloro-3-fluoropropane, 1-chloro-4-fluorobutane, 2-bromo-2-methylpropane, 2-chloro-2-methylpropane, 2-bromo-2-methylbutane, 2,3-dichloro-2-methylbutane, 1-iodobutane, 1,1,2-trichloropropane, 1,3-dichlorobutane, 2,3-dichlorobutane, 1,2,2-trichloropropane, cis-1,3-dichloropropene, trans-1,3-dichloropropene, 1,3-dichloro-trans-2-butene, 1,2-dichloro-2-butene, 2-chloro-2-methyl-butane.

De préférence, les alcools sont sélectionnés parmi le groupe consistant en Methanol, ethanol, 2-propanol, 2,2-dimethyl-1-propanol, 2,2,2-trifluoroethanol, tert-butanol, 2,2,3,3-tetraflouro-1-propanol, 2-chloro-1-propanol, propanol, 2-allyloxyethanol, 2-butanol, 2-aminophenol, 2-methyl-2-butanol, 2-ethyl-1-butanol, isobutanol, 3-pentanol, 1-butanol, 1-methoxy2-propanol, 1-(dimethylamino)-2-propanol, 2-methyl-1-pentanol, 3-methyl-3-pentanol, 2-methoxy1-propanol, 1-ethoxy-2-propanol, 4-methyl-2-pentanol, 2-chloroethanol, 1,2-octanediol, 2-(dimethylamino)-ethanol, 3-hexanol, 2-hexanol, 2-ethoxy-1-propanol, 1-pentanol, 2-propoxyethanol, 1-propoxy-2-propanol, 2,2-difluoroethanol, 1,1,1-trifluoro-2-propanol, 4,4,4-trifluorobutanol, 3-fluoropropanol, 2,3-dimethylbutanol, 1-chloro-2-methyl-2-propanol.

De préférence, les cétones sont sélectionnées parmi le groupe consistant en propanone, butanone, 3-pentanone, 2-pentanone, 3,3-dimethyl-2-butanone, 4-methyl-2-pentanone, 2-hexanone, 5-hexen-2-one, 4-methyl-2-hexanone, 1,1,1-trifluoro-2-propanone.

De préférence, les amines sont sélectionnées parmi le groupe consistant en éthylamine, isopropylamine, ethylmethylamine, 2-amino-2-methylpropane, n-propylamine, isopropylmethylamine, diethylamine, 2-butanamine, n-methylpropylamine, 1-butylamine, diisopropylamine, 3-methyl-2-butanamine, 3-pentylamine, n-methylbutylamine, 1-methoxy-2-propanamine, 2-methoxyethanamine, 2-methoxy-1propanamine, n-pentylamine, n-methylhydroxylamine, dipropylamine, 2-ethoxyethanamine, n-methyl-1,2-ethanediamine, pyridine, 1,2-diaminoethane, 1,2-propanediamine, 2-ethylbutylamine, n-ethylethylenediamine, 2-methylpyridine, 4-methyl-2-hexanamine, hexylamine, cyclohexylamine, n-ethyl-2-dimethylaminoethylamine, 1,3-propanediamine, 2-heptanamine, n,n-diethylethylenediamine, 2,6-dimethylpyridine, 4-methylpyridine, n,n'-diethyl-1,2-ethanediamine, dimethylethanolamine, 1,1-diethoxy-n,n-dimethylmethanamine.

De préférence, les esters sont sélectionnés parmi le groupe consistant méthylacétate, ethylacetate, n-propylformate, isopropylacetate, tert-butylacetate, ethylpropionate, sec-butylacetate, diethylcarbonate, n-butylacetate, bromoaceticacidmethylester, methylformate, methylhexanoate, isopropylformate.

De préférence, les éthers sont sélectionnés parmi le groupe consistant en diethylether, 2-ethoxy-propane, methyl-t-butylether, 2,2-dimethoxypropane, 1-ethoxy-2-methylpropane, 1,2-dimethoxyethane, diethoxymethane, 1-ethoxybutane, 1-methoxy-pentane, 1,2-dimethoxypropane, 1,1-diethoxyethane, trimethoxymethane, 2-chloro-1,1-dimethoxyethane, 2,2-diethoxypropane, 1,1-diethoxypropane, 2-methoxyethanol, methoxycyclohexane, chloromethoxymethane, ethoxyethanol, di-n-butylether, diisopropylether, 1-ethoxy-hexane, 1,1,1-triethoxyethane, 1-methoxy-2-acetoxypropane, dimethoxymethane, ethoxy-ethene, di-n-propylether, 2-methoxy-1-propene, 2,2,2-trifluoroethylmethylether, methylcyclopropylether, 2-ethoxy-2-methyl-propane, 2-ethoxy-butane, sec-butyl-tert-butylether, isobutyl-tert-butylether, 1-methoxy-2-methyl-butane, isopropyl-isobutyl-ether.

De préférence, les aldéhydes sont sélectionnés parmi le groupe consistant en acétaldehyde, isobutanal, methylglyoxal, 2-methylbutanal, 2,6-dimethyl-5-heptenal, hexanal, ethanedial.

De préférence, les nitriles sont sélectionnés parmi le groupe consistant en acetonitrile, propionitrile, butyronitrile, valeronitrile, (methyleneamino)acetonitrile.

De préférence, le carbonate est le diéthylcarbonate.

De préférence, les amides incluent l'éthanethioamide.

De préférence, les thioalkyles sont sélectionnés parmi le group consistant en éthanethiol, dimethylsulfide, 2-propanethiol, tert-butylthiol, 3-mercapto-1,2-propanediol, 1-propanethiol, butanethiol, tetrahydrothiophene, 1-pentanethiol, diethylsulfide, 2-butanethiol, 2-methyl-1-propanethiol, 4-methoxy-2-methyl-2-butanethiol.

De préférence, les hétérocycles sont choisis parmi le groupe consistant en n-ethyl-morpholine, 1-methylpiperazine, n-methylmorpholine, 2-methylpyrazine, tetrahydrofurane, 1,3,5-trioxane, dioxane, 1,3-dioxane, piperidine, 2,6-dimethylmorpholine. Le dioxane se réfère au 1,4-dioxane.

Ledit agent d'extraction organique décrit peut être éthylamine, bromofluoromethane, 1-bromo-1,2-difluoroethylene, acetaldehyde, 1,1,1-trifluoro-2-propanone, 1,1,1-trifluoro-2-bromoethane, 2,2,2-trifluoroethylmethylether, isopropylamine, methylformate, 2-methoxy-1-propene, diethylether, 1,2-epoxypropane, ethanethiol, ethoxy-ethene, ethylmethylamine, dimethylsulfide, 2-chloropropane, bromoethane, dimethoxymethane, iodomethane, 2-amino-2-methylpropane, methylcyclopropylether, n-propylamine, isopropylmethylamine, ethanedial, 2-chloro-2-methylpropane, 2-propanethiol, 2-ethoxy-propane, methyl-t-butylether, diethylamine, propanone, methylacetate, 4-methoxy-2-methyl-2-butanethiol, 2-bromopropane, chloromethoxymethane, 2-butanamine, n-methylpropylamine, tert-butylthiol, isobutanal, methanol, tetrahydrofurane, 1-propanethiol, chlorobromomethane, isopropylformate, diisopropylether, 3-bromopropene, 1-bromopropane, methylglyoxal, iodoethane, 2-ethoxy-2-methyl-propane, 2-bromo-2-methylpropane, 2,2,2-trifluoroethanol, 1-chloro-3-fluoropropane, 1,1,1-trifluoro-2-propanol, 1-butylamine, ethylacetate, ethanol, butanone, n-propylformate, 2-ethoxy-butane, 2-propanol, acetonitrile, tert-butanol, 1-methoxy-2-methyl-butane, 2,2-dimethoxypropane, 2-chloro-2-methylbutane, 1,2-dichloroethane, 1-ethoxy-2-methylpropane, diisopropylamine, 2-butanethiol, 1,2-dimethoxyethane, 3-methyl-2-butanamine, diethoxymethane, 2-methyl-1-propanethiol, isopropylacetate, 2-iodopropane, di-n-propylether, 3-pentylamine, n-methylbutylamine, 2-bromobutane, diethylsulfide, 1-ethoxybutane, 1-methoxy-2-propanamine, 2-methylbutanal, 2-methoxyethanamine, 2,2-difluoroethanol, 1,2-dichloropropane, propanol, tert-butylacetate, propionitrile, trichloroacetaldehyde, 2-allyloxyethanol, butanethiol, 1-methoxy-pentane, ethylpropionate, 2-butanol, 1,2-dimethoxypropane, isopropyl-isobutyl-ether, 1-chloro-4-fluorobutane, 1-bromo-3-fluoropropane, dioxane, 1-bromobutane, 3-pentanone, 1,1-diethoxyethane, 2-pentanone, 2-methyl-2-butanol, 1-iodopropane, 2-methoxy-1propanamine, trimethoxymethane, cis-1,3-dichloropropene, n-pentylamine, 3,3-dimethyl-2-butanone, 1,3-dioxane, piperidine, 1-bromo-2-chloroethane, isobutanol, 2-bromo-2-methylbutane, dipropylamine, 2,2,3,3-tetraflouro-1-propanol, 2-ethoxyethanamine, triethylfluorosilane, sec-butylacetate, trans-1,3-dichloropropene, 2,2-dimethyl-1-propanol, n-methyl-1,2-ethanediamine, 2,2-diethoxypropane, 1,3,5-trioxane, pyridine, n-methylmorpholine, 3-pentanol, 4-methyl-2-pentanone, 1,2-diaminoethane, isobutyl-tert-butylether, 2-bromopentane, butyronitrile, 1-butanol, 2,3-dichlorobutane, sec-butyl-tert-butylether, 1-methoxy2-propanol, 1,2-propanediamine, 2,6-dimethyl-5-heptenal, 1-bromo-3-methylbutane, 1,3-dichloro-trans-2-butene, 1,3-dichloropropane, 1-(dimethylamino)-2-propanol, tetrahydrothiophene, 3-methyl-3-pentanol, 1,2-dibromo-1-fluoroethane, 1,1-diethoxypropane, 1,2,2-trichloropropane, 1-chloro-2-methyl-2-propanol, 2-methoxyethanol, 4,4,4-trifluorobutanol, 2-ethylbutylamine, diethylcarbonate, n-butylacetate, 1-pentanethiol, 2-chloro-1,1-dimethoxyethane, 2-hexanone, n-ethylethylenediamine, 3-fluoropropanol, 5-hexen-2-one, 2,3-dichloro-2-methylbutane, 1,1-diethoxy-n,n-dimethylmethanamine, 2-methylpyridine, 1-bromopentane, 2-methoxy1-propanol, 1,2-dichloro-2-butene, 1-iodobutane, hexanal, 1-ethoxy-2-propanol, 1,2-dibromoethane, 4-methyl-2-pentanol, bromoaceticacidmethylester, 1,1,2-trichloropropane, 1,2-octanediol, 4-methyl-2-hexanamine, hexylamine, 2-chloro-1-propanol, methoxycyclohexane, 2-(dimethylamino)-ethanol, 1,3-dichlorobutane, cyclohexylamine, n-ethyl-2-dimethylaminoethylamine, ethoxyethanol, 3-hexanol, 2-hexanol, 2-methylpyrazine, 2-ethoxy-1-propanol, 1-pentanol, n-ethyl-morpholine, 1-methylpiperazine, 1,3-propanediamine, di-n-butylether, valeronitrile, (methyleneamino)acetonitrile, 1,2-dibromopropane, 1,2,3-trichloropropene, 2-heptanamine, 2,3-dimethylbutanol, 1-ethoxy-hexane, 1-chloro-3-bromopropane, n,n-diethylethylenediamine, 3-furfural, 2,6-dimethylpyridine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, 2-ethyl-1-butanol, 2-methyl-1-pentanol, methylhexanoate, 2-propoxyethanol, 1-propoxy-2-propanol, dimethylethanolamine.

L'agent d'extraction organique à utiliser peut être sélectionné en fonction des composés présents dans ladite première composition. Ainsi, l'agent d'extraction organique peut être sélectionné en fonction du facteur de séparation et de la capacité d'absorption établi pour une composition particulière. Outre ces deux critères, le choix de l'agent d'extraction organique peut se baser optionnellement sur d'autres critères commerciaux ou environnementaux, tels que par exemple le coût de l'agent d'extraction organique, sa disponibilité sur le marché, ses propriétés toxiques ou inflammable. De plus, selon un mode de réalisation particulier, afin d'optimiser le fonctionnement des colonnes de distillation utilisées à l'étape b) et c) du présent procédé de purification du 1,1,1,2,2-pentachloropropane, le point d'ébullition de l'agent d'extraction organique peut être de 0°C à 200°C, avantageusement de 10°C à 190°C, de préférence de 10°C à 180°C, en particulier de 10°C à 150°C, plus particulièrement de 20°C à 150°C, de manière privilégiée de 50°C à 150°C, de manière encore plus privilégiée de 75 à 150°C.

Selon un mode de réalisation préféré, ledit agent d'extraction organique a un facteur de séparation $S_{1,2}$ supérieur ou égal à 1,1, ledit facteur de séparation étant calculé par la formule $S_{1,2} = (\gamma_{1,S}*P1)/(\gamma_{2,S}*P2)$ dans laquelle

$\gamma_{1,S}$ représente le coefficient d'activité du 1,1,1,2,2-pentafluoropropane dans ledit agent d'extraction organique à dilution infinie,

P1 représente la pression de vapeur saturante du 1,1,1,2,2-pentafluoropropane, $\gamma_{2,S}$ représente le coefficient d'activité dudit au moins un des composés consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) dans ledit agent d'extraction organique à dilution infinie,

P2 représente la pression de vapeur saturante de dudit au moins un des composés consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf).

Avantageusement, le facteur de séparation $S_{1,2}$ est supérieur ou égal à 1,2, de préférence supérieur ou égal à 1,4, plus préférentiellement supérieur ou égal à 1,5, en particulier supérieur ou égal à 1,8, plus particulièrement supérieur ou égal à 1,9.

Selon un mode de réalisation préféré, ledit agent d'extraction organique a une capacité d'absorption $C_{2,S}$ supérieure ou égale à 0,20, ladite capacité d'absorption étant calculé par la formule $C_{2,S} = 1/(\gamma_{2,s})$ dans laquelle $\gamma_{2,S}$ représente le coefficient d'activité dudit au moins un des composés consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) dans ledit agent d'extraction organique à dilution infinie. Avantageusement, la capacité d'absorption $C_{2,S}$ est supérieure ou égale à 0,40, de préférence supérieure ou égale à 0,60, plus préférentiellement supérieure ou égale à 0,80, en particulier supérieure ou égale à 0,90, plus particulièrement supérieure ou égale à 1,0, de manière privilégiée supérieure ou égale à 1,05.

Selon un mode de réalisation préféré, ledit agent d'extraction organique peut avoir un facteur de séparation $S_{1,2}$ supérieur ou égal à 1,1, avantageusement supérieur ou égal à 1,2, de préférence supérieur ou égal à 1,4, plus préférentiellement supérieur ou égal à 1,5, en particulier supérieur ou égal à 1,8 et plus particulièrement supérieur ou égal à 1,9 ; et une capacité d'absorption $C_{2,S}$ supérieure à 0,20, avantageusement supérieure ou égale à 0,4, de préférence supérieure ou égale à 0,60, plus préférentiellement supérieure ou égale à 0,80, en particulier supérieure ou égale à 0,90, plus particulièrement supérieure ou égale à 1,0, de manière privilégiée supérieure ou égale à 1,05.

Ladite première composition peut être une composition azéotropique ou quasi-azéotropique comprenant 1,1,1,2,2-pentafluoropropane (245cb), trans 1,3,3,3-tetrafluoro-1-propene (1234ze-E) et optionnellement au moins un des composés choisi parmi le groupe consistant en 1,1-difluoroéthane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf).

Ladite première composition peut être une composition azéotropique ou quasi-azéotropique comprenant 1,1,1,2,2-pentafluoropropane (245cb), trans-1,3,3,3-tetrafluoro-1-propene (1234ze-E) et optionnellement au moins deux, au moins trois, au moins quatre, au moins cinq, au moins six ou l'ensemble des composés choisi parmi le groupe consistant en 1,1-difluoroéthane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf). La teneur en chacun des composés dans la première composition azéotropique ou quasi-azéotropique est telle qu'exprimée ci-dessus.

En fonction du ou des composés à éliminer dans ladite première composition, ledit facteur de séparation et ladite capacité d'absorption pourront être calculés pour un couple binaire particulier consistant en 1,1,1,2,2-pentafluoropropane (245cb), trans-1,3,3,3-tetrafluoro-1-propene (1234ze-E) et un des composés choisi parmi le groupe consistant en 1,1-difluoroéthane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf). Ainsi, pour sélectionner ledit agent d'extraction organique apte à être utilisé dans l'étape b) de distillation extractive, le facteur de séparation et la capacité d'absorption peuvent être calculés par exemple pour un couple binaire 1,1,1,2,2-pentafluoropropane (245cb) et trans-1,3,3,3-tetrafluoro-1-propene (1234ze-E).

Le facteur de séparation $S_{1,2}$ permet de déterminer la capacité d'un agent d'extraction organique à séparer deux ou plusieurs composés. La capacité d'absorption $C_{2,S}$ permet de déterminer la quantité de solvant à utiliser pour obtenir la séparation entre les composés considérés. Pour l'ensemble des premières compositions détaillées ci-dessous, ledit agent d'extraction organique peut avoir un facteur de séparation $S_{1,2}$ supérieur ou égal à 1,1, avantageusement supérieur ou égal à 1,2, de préférence supérieur ou égal à 1,4, plus préférentiellement supérieur ou égal à 1,5, en particulier supérieur ou égal à 1,8 et plus particulièrement supérieur ou égal à 1,9 ; et/ou ledit agent d'extraction organique peut avoir une capacité d'absorption $C_{2,S}$ supérieure ou égale à 0,20, avantageusement supérieure ou égale à 0,4, de préférence supérieure ou égale à 0,5, plus préférentiellement supérieure ou égale à 0,8, en particulier supérieure ou égale à 0,9, plus particulièrement supérieure ou égale à 1,0, de manière privilégiée supérieure ou égale à 1,05.

En particulier, ladite première composition peut être une composition azéotropique ou quasi-azéotropique comprenant 1,1,1,2,2-pentafluoropropane (245cb) et trans-1,3,3,3-tetrafluoro-1-propene (1234ze-E). La teneur en chacun des composés dans cette composition particulière est telle qu'exprimée ci-dessus en référence aux teneurs individuelles de chacun des composés. Ladite composition azéotropique ou quasi-azéotropique est obtenue à une température de -30°C à 50°C. Ladite composition azéotropique ou quasi-azéotropique est obtenue pour une pression de 0,5 bar à 10 bars. Ladite composition azéotropique ou quasi-azéotropique peut être obtenue pour une teneur en poids de 1,1,1,2,2-pentafluoropropane (245cb) sur base du poids total de la composition azéotropique ou quasi-azéotropique. Ainsi, la seconde composition peut comprendre 1,1,1,2,2-pentafluoropropane (245cb), trans-1,3,3,3-tetrafluoro-1-propene (1234ze-E) et ledit agent d'extraction organique. Ledit agent d'extraction organique peut avoir un facteur de séparation $S_{1,2}$ supérieur ou égal à 1,5, ledit facteur de séparation étant calculé par la formule $S_{1,2} = (\gamma_{1,S}{*}P1)/(\gamma_{2,S}{*}P2)$ dans laquelle $y_{i,s}$ représente le coefficient d'activité du 1,1,1,2,2-pentafluoropropane par rapport audit agent d'extraction organique, P1 représente la pression de vapeur saturante du 1,1,1,2,2-pentafluoropropane, $\gamma_{2,S}$ représente le coefficient d'activité du trans-1,3,3,3-tetrafluoro-1-propene dans ledit agent d'extraction organique à dilution infinie, P2 représente la pression de vapeur saturante du trans-1,3,3,3-tetrafluoro-1-propene. Ledit agent d'extraction organique peut avoir une capacité d'absorption $C_{2,S}$ supérieure ou égale à 0,6, ladite capacité d'absorption étant calculé par la formule $C_{2,S} = 1/(\gamma_{2,s})$ dans laquelle $\gamma_{2,S}$ représente le coefficient d'activité dans ledit agent d'extraction organique à dilution infinie. Ainsi, pour séparer la première composition azéotropique ou quasi-azéotropique comprenant 1,1,1,2,2-pentafluoropropane (245cb) et trans-1,3,3,3-tetrafluoro-1-propene (1234ze-E), un agent d'extraction organique ayant un facteur de séparation $S_{1,2}$ supérieur ou égal à 1,5 et une capacité d'absorption $C_{2,S}$ supérieure ou égale à 0,6 peut être utilisé ; ledit agent d'extraction organique peut ainsi être éthylamine, acetaldehyde, isopropylamine, methylformate, diethylether, 1,2-epoxypropane, ethylmethylamine, dimethoxymethane, 2-amino-2-methylpropane, methylcyclopropylether, n-propylamine, isopropylmethylamine, 2-ethoxy-propane, methyl-t-butylether, diethylamine, propanone, methylacetate, 4-methoxy-2-methyl-2-butanethiol, 2-butanamine, n-methylpropylamine, isobutanal, tetrahydrofurane, isopropylformate, diisopropylether, 2-ethoxy-2-methyl-propane, 1-butylamine, ethylacetate, butanone, n-propylformate, 2-ethoxy-butane, 1-methoxy-2-methyl-butane, 2,2-dimethoxypropane, 1-ethoxy-2-methylpropane, diisopropylamine, 1,2-dimethoxyethane, 3-methyl-2-butanamine, diethoxymethane, isopropylacetate, di-n-propylether, 3-pentylamine, n-methylbutylamine, 1-ethoxybutane, 1-methoxy-2-propanamine, 2-methylbutanal, 2-methoxyethanamine, tert-butylacetate, propionitrile, 2-allyloxyethanol, 1-methoxy-pentane, ethylpropionate, 1,2-dimethoxypropane, dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, dipropylamine, 2-ethoxyethanamine, sec-butylacetate, n-methyl-1,2-ethanediamine, 2,2-diethoxypropane, pyridine, 4-methyl-2-pentanone, 1,2-diaminoethane, butyronitrile, sec-butyl-tert-butylether, 1-methoxy2-propanol, 1,2-propanediamine, 2,6-dimethyl-5-heptenal, 1-(dimethylamino)-2-propanol, 3-methyl-3-pentanol, 1,1-diethoxypropane, 2-ethylbutylamine, diethylcarbonate, n-butylacetate, 2-hexanone, n-ethylethylenediamine, 5-hexen-2-one, 2-methylpyridine, 2-methoxy1-propanol, hexanal, 1-ethoxy-2-propanol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 2-methylpyrazine, 2-ethoxy-1-propanol, 1-methylpiperazine, 1,3-propanediamine, di-n-butylether, valeronitrile, 2-heptanamine, 1-ethoxy-hexane, n,n-diethylethylenediamine, 2,6-dimethylpyridine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol.

Avantageusement, ledit agent d'extraction organique peut avoir un facteur de séparation $S_{1,2}$ supérieur ou égal à 1,8 et/ou une capacité d'absorption $C_{2,S}$ supérieure ou égale à 0,8. Ledit agent d'extraction organique peut ainsi être Ethylamine, isopropylamine, ethylmethylamine, 2-amino-2-methylpropane, n-propylamine, isopropylmethylamine, diethylamine, propanone, 2-butanamine, n-methylpropylamine, tetrahydrofurane, 1-butylamine, ethylacetate, butanone, 1,2-dimethoxyethane, 3-methyl-2-butanamine, 3-pentylamine, n-methylbutylamine, 1-methoxy-2-propanamine, 2-methoxyethanamine, ethylpropionate, dioxane, 3-pentanone, 2-pentanone, 2-methoxy-1propanamine, trimethoxymethane, n-pentylamine, 3,3-dimethyl-2-butanone, 1,3-dioxane, piperidine, 2-ethoxyethanamine, n-methyl-1,2-ethanediamine, 1,2-diaminoethane, butyronitrile, 1-methoxy2-propanol, 1,2-propanediamine, 1-(dimethylamino)-2-propanol, 2-ethylbutylamine, diethylcarbonate, n-butylacetate, 2-hexanone, n-ethylethylenediamine, 2-methoxy1-propanol, 1-ethoxy-propanol 4-methyl-2-hexanamine, hexylamine, methoxycyclohexane, 2-(dimethylamino)-ethanol, cyclohexylamine, n-ethyl-2-dimethylaminoethylamine, 2-ethoxy-1-propanol, 1-methylpiperazine, 1,3-propanediamine, valeronitrile, 2-heptanamine, n,n-diethylethylenediamine, 4-methyl-2-hexanone, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol.

De préférence, ledit agent d'extraction organique peut avoir un facteur de séparation $S_{1,2}$ supérieur ou égal à 1,9 et/ou une capacité d'absorption $C_{2,S}$ supérieure ou égale à 0,9. Ledit agent d'extraction organique peut ainsi être éthylamine, isopropylamine, ethylmethylamine, 2-amino-2-methylpropane, n-propylamine, isopropylmethylamine, diethylamine, propanone, 2-butanamine, n-methylpropylamine, tetrahydrofurane, 1-butylamine, ethylacetate, butanone, 1,2-dimethoxyethane, 3-methyl-2-butanamine, 3-pentylamine, n-methylbutylamine, 1-methoxy-2-propanamine, 2-methoxyethanamine, ethylpropionate, dioxane, 3-pentanone, 2-pentanone, 2-methoxy-1propanamine, n-pentylamine, 3,3-dimethyl-2-butanone, 1,3-dioxane, piperidine, 2-ethoxyethanamine, n-methyl-1,2-ethanediamine, 1,2-diaminoethane, 1,2-propanediamine, 1-(dimethylamino)-2-propanol, 2-ethylbutylamine, n-butylacetate, 2-hexanone, n-ethylethylenediamine, 1-ethoxy-2-propanol, 4-methyl-2-hexanamine, hexylamine, 2-(dimethylamino)-ethanol, cyclohexylamine, n-ethyl-2-dimethylaminoethylamine, 2-ethoxy-1-propanol, 1-methylpiperazine, 1,3-propanediamine, 2-heptanamine, n,n-diethylethylenediamine, 1-methoxy-2-acetoxypropane, 4-methylpyridine, n,n'-diethyl-1,2-ethanediamine, 2,6-dimethyl-morpholine, methylhexanoate, 1-propoxy-2-propanol.

De préférence, ledit agent d'extraction organique est choisi parmi le groupe consistant en Ethylamine, isopropylamine, n-propylamine, diethylamine, propanone, tetrahydrofurane, ethylacetate, butanone, 3-pentylamine, 2-methoxyethanamine, dioxane, 3-pentanone, 2-pentanone, n-pentylamine, 1,3-dioxane, 1,2-diaminoethane, 1,2-propanediamine, 2-methoxyethanol, n-butylacetate, 1-ethoxy-2-propanol. En particulier, ledit agent d'extraction organique est choisi parmi le groupe consistant en éthylamine, isopropylamine, n-propylamine, diethylamine, tetrahydrofurane, ethylacetate, butanone, 3-pentylamine, 2-methoxyethanamine, dioxane, 2-pentanone, n-pentylamine, 1,3-dioxane, 1,2-diaminoethane, 1,2-propanediamine, 2-methoxyethanol, n-butylacetate, 1-ethoxy-2-propanol. Plus particulièrement, ledit agent d'extraction organique est choisi parmi le groupe consistant en éthylamine, isopropylamine, n-propylamine, diethylamine, tetrahydrofurane, ethylacetate, butanone, 3-pentylamine, 2-methoxyethanamine, dioxane, 2-pentanone, n-pentylamine, 1,3-dioxane, 1,2-diaminoethane, 1,2-propanediamine, 1-ethoxy-2-propanol.

Également décrite est une première composition qui est une composition azéotropique ou quasi-azéotropique comprenant 1,1,1,2,2-pentafluoropropane (245cb) et cis-1,3,3,3-tetrafluoro-1-propene (1234ze-Z). La teneur en chacun des composés dans cette composition particulière est telle qu'exprimée ci-dessus en référence aux teneurs individuelles de chacun des composés. Ainsi, la seconde composition peut comprendre 1,1,1,2,2-pentafluoropropane (245cb), cis-1,3,3,3-tetrafluoro-1-propene (1234ze-Z) et ledit agent d'extraction organique, ayant de préférence un facteur de séparation $S_{1,2}$ supérieur ou égal à 1,1, avantageusement supérieur ou égal à 1,2, de préférence supérieur ou égal à 1,4, plus préférentiellement supérieur ou égal à 1,5, en particulier supérieur ou égal à 1,8 et plus particulièrement supérieur ou égal à 1,9 ; et/ou ledit agent d'extraction organique peut avoir une capacité d'absorption $C_{2,S}$ supérieure ou égale à 0,20, avantageusement supérieure ou égale à 0,4, de préférence supérieure ou égale à 0,5, plus préférentiellement supérieure ou égale à 0,8, en particulier supérieure ou égale à 0,9, plus particulièrement supérieure ou égale à 1,0, de manière privilégiée supérieure ou égale à 1,05. Ledit agent d'extraction organique peut être tel que défini ci-dessus pour la première composition comprenant 1,1,1,2,2-pentafluoropropane (245cb) et trans-1,3,3,3-tetrafluoro-1-propene (1234ze-E).

Également décrite est une première composition qui est une composition azéotropique ou quasi-azéotropique comprenant 1,1,1,2,2-pentafluoropropane (245cb) et 1,1-difluoroéthane (152a). La teneur en chacun des composés dans cette composition particulière est telle qu'exprimée ci-dessus en référence aux teneurs individuelles de chacun des composés. Ainsi, la seconde composition peut comprendre 1,1,1,2,2-pentafluoropropane (245cb), 1,1-difluoroéthane (152a) et ledit agent d'extraction organique, ayant de préférence un facteur de séparation $S_{1,2}$ supérieur ou égal à 1,1, avantageusement supérieur ou égal à 1,2, de préférence supérieur ou égal à 1,4, plus préférentiellement supérieur ou égal à 1,5, en particulier supérieur ou égal à 1,8 et plus particulièrement supérieur ou égal à 1,9 ; et/ou ledit agent d'extraction organique peut avoir une capacité d'absorption $C_{2,S}$ supérieure ou égale à 0,20, avantageusement supérieure ou égale à 0,4, de préférence supérieure ou égale à 0,5, plus préférentiellement supérieure ou égale à 0,8, en particulier supérieure ou égale à 0,9. Ledit agent d'extraction organique peut être tel que défini ci-dessus pour la première composition comprenant 1,1,1,2,2-pentafluoropropane (245cb) et trans-1,3,3,3-tetrafluoro-1-propene (1234ze-E).

Également décrite est une première composition qui est une composition azéotropique ou quasi-azéotropique comprenant 1,1,1,2,2-pentafluoropropane (245cb) et 1,1,1,2-tetrafluoroethane (134a). La teneur en chacun des composés dans cette composition particulière est telle qu'exprimée ci-dessus en référence aux teneurs individuelles de chacun des composés. Ainsi, la seconde composition peut comprendre 1,1,1,2,2-pentafluoropropane (245cb), 1,1,1,2-tetrafluoroethane (134a) et ledit agent d'extraction organique, ayant de préférence un facteur de séparation $S_{1,2}$ supérieur ou égal à 1,1, avantageusement supérieur ou égal à 1,2, de préférence supérieur ou égal à 1,4, plus préférentiellement supérieur ou égal à 1,5, en particulier supérieur ou égal à 1,8 et plus particulièrement supérieur ou égal à 1,9 ; et/ou ledit agent d'extraction organique peut avoir une capacité d'absorption $C_{2,S}$ supérieure ou égale à 0,20, avantageusement supérieure ou égale à 0,4, de préférence supérieure ou égale à 0,5, plus préférentiellement supérieure ou égale à 0,8, en particulier supérieure ou égale à 0,9. Ledit agent d'extraction organique peut être tel que défini ci-dessus pour la première composition comprenant 1,1,1,2,2-pentafluoropropane (245cb) et trans-1,3,3,3-tetrafluoro-1-propene (1234ze-E).

Également décrite est une première composition qui est une composition azéotropique ou quasi-azéotropique comprenant 1,1,1,2,2-pentafluoropropane (245cb) et trans-1,2,3,3,3-pentafluoropropene (1225ye-E). La teneur en chacun des composés dans cette composition particulière est telle qu'exprimée ci-dessus en référence aux teneurs individuelles de chacun des composés. Ainsi, la seconde composition peut comprendre 1,1,1,2,2-pentafluoropropane (245cb), trans-1,2,3,3,3-pentafluoropropene (1225ye-E) et ledit agent d'extraction organique, ayant de préférence un facteur de séparation $S_{1,2}$ supérieur ou égal à 1,1, avantageusement supérieur ou égal à 1,2, de préférence supérieur ou égal à 1,4, plus préférentiellement supérieur ou égal à 1,5, en particulier supérieur ou égal à 1,8 et plus particulièrement supérieur ou égal à 1,9 ; et/ou ledit agent d'extraction organique peut avoir une capacité d'absorption $C_{2,S}$ supérieure ou égale à 0,20, avantageusement supérieure ou égale à 0,4, de préférence supérieure ou égale à 0,5, plus préférentiellement supérieure ou égale à 0,8, en particulier supérieure ou égale à 0,9. Ledit agent d'extraction organique peut être tel que défini ci-dessus pour la première composition comprenant 1,1,1,2,2-pentafluoropropane (245cb) et trans-1,3,3,3-tetrafluoro-1-propene (1234ze-E).

Également décrite est une première composition qui est une composition azéotropique ou quasi-azéotropique comprenant 1,1,1,2,2-pentafluoropropane (245cb) et cis-1,2,3,3,3-pentafluoropropene (1225ye-Z). La teneur en chacun des composés dans cette composition particulière est telle qu'exprimée ci-dessus en référence aux teneurs individuelles de chacun des composés. Ainsi, la seconde composition peut comprendre 1,1,1,2,2-pentafluoropropane (245cb), cis-1,2,3,3,3-pentafluoropropene (1225ye-Z) et ledit agent d'extraction organique, ayant de préférence un facteur de séparation $S_{1,2}$ supérieur ou égal à 1,1, avantageusement supérieur ou égal à 1,2, de préférence supérieur ou égal à 1,4, plus préférentiellement supérieur ou égal à 1,5, en particulier supérieur ou égal à 1,8 et plus particulièrement supérieur ou égal à 1,9 ; et/ou ledit agent d'extraction organique peut avoir une capacité d'absorption $C_{2,S}$ supérieure ou égale à 0,20, avantageusement supérieure ou égale à 0,4, de préférence supérieure ou égale à 0,5, plus préférentiellement supérieure ou égale à 0,8, en particulier supérieure ou égale à 0,9. Ledit agent d'extraction organique peut être tel que défini ci-dessus pour la première composition comprenant 1,1,1,2,2-pentafluoropropane (245cb) et trans-1,3,3,3-tetrafluoro-1-propene (1234ze-E).

Également décrite est une première composition qui est une composition azéotropique ou quasi-azéotropique comprenant 1,1,1,2,2-pentafluoropropane (245cb) et 3,3,3-trifluoropropene (1243zf). La teneur en chacun des composés dans cette composition particulière est telle qu'exprimée ci-dessus en référence aux teneurs individuelles de chacun des composés. Ainsi, la seconde composition peut comprendre 1,1,1,2,2-pentafluoropropane (245cb), 3,3,3-trifluoropropene (1243zf) et ledit agent d'extraction organique, ayant de préférence un facteur de séparation $S_{1,2}$ supérieur ou égal à 1,1, avantageusement supérieur ou égal à 1,2, de préférence supérieur ou égal à 1,4, plus préférentiellement supérieur ou égal à 1,5, en particulier supérieur ou égal à 1,8 et plus particulièrement supérieur ou égal à 1,9 ; et/ou ledit agent d'extraction organique peut avoir une capacité d'absorption $C_{2,S}$ supérieure ou égale à 0,20, avantageusement supérieure ou égale à 0,4, de préférence supérieure ou égale à 0,5, plus préférentiellement supérieure ou égale à 0,8, en particulier supérieure ou égale à 0,9. Ledit agent d'extraction organique peut être tel que défini ci-dessus pour la première composition comprenant 1,1,1,2,2-pentafluoropropane (245cb) et trans-1,3,3,3-tetrafluoro-1-propene (1234ze-E).

Selon un mode de réalisation préféré, ladite troisième composition comprenant le l'agent d'extraction organique, trans-1,3,3,3-tetrafluoro-1-propene (1234ze-E) et optionnellement ledit au moins un des composés choisi parmi le groupe consistant en 1,1-difluoroéthane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) peut être soumis à une distillation pour séparer l'agent d'extraction organique et ledit au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf).

Selon un mode de réalisation particulier, le courant comprenant le 1,1,1,2,2-pentafluoropropane séparé à l'étape b) du présent procédé est récupéré pour être utilisé dans un procédé de production du 2,3,3,3-tetrafluoropropène. De préférence, dans ce mode de réalisation, des traces d'agent d'extraction organique peuvent être présentes dans le courant comprenant le 1,1,1,2,2-pentafluoropropane. De préférence, l'agent d'extraction organique est sélectionné de sorte à ne pas altérer les performances du catalyseur utilisé dans le procédé de production du 2,3,3,3-tetrafluoropropène.

Le présente procédé permet donc de purifier le 1,1,1,2,2-pentafluoropropane. Avantageusement, la teneur en au moins un des composés sélectionné parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) dans le courant comprenant du 1,1,1,2,2-pentafluoropropane, obtenu à l'étape b) du présent procédé de purification, est inférieure à la teneur de celui-ci ou de ceux-ci dans ladite première composition. Par exemple, la teneur en l'un quelconque des composés peut être diminuée de 50%, avantageusement de 75%, de préférence de 90%, en particulier de 95%, plus particulièrement de 98%. Avantageusement, la teneur en au moins deux, au moins trois, au moins quatre, au moins cinq ou en l'ensemble desdits composés sélectionné parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) peut être diminuée de 50%, avantageusement de 75%, de préférence de 90%, en particulier de 95%, plus particulièrement de 98%. De préférence, le courant comprenant le 1,1,1,2,2-pentafluoropropane obtenu à l'étape b) du présent procédé de purification peut être dépourvu d'au moins un, d'au moins deux, d'au moins trois, d'au moins quatre, d'au moins cinq ou de l'ensemble des composés sélectionné parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) lorsque celui-ci ou ceux-ci sont présents dans ladite première composition. En particulier, le courant comprenant le 1,1,1,2,2-pentafluoropropane obtenu à l'étape b) du présent procédé de purification peut être dépourvu de trans-1,3,3,3-tetrafluoro-1-propene (1234ze-E). Le terme « dépourvu » signifie que le courant comprenant le 1,1,1,2,2-pentafluoropropane comprenant moins de 50 ppm, avantageusement moins de 20 ppm, de préférence moins de 10 ppm du composé considéré sur base du poids total du courant.

Selon un mode de réalisation préféré, ladite première composition mise en oeuvre à l'étape a) du présent procédé de purification est obtenue à partir d'une composition de 2,3,3,3-tetrafluoro-1-propene (1234yf). La composition de 2,3,3,3-tetrafluoro-1-propene (1234yf) peut être préalablement purifié avant d'être utilisé dans le présent procédé de purification du 1,1,1,2,2-pentafluoropropane. Ainsi, le présent procédé peut comprendre préalablement à l'étape a) les étapes :

i') mise en oeuvre d'une composition A comprenant 2,3,3,3-tetrafluoro-1-propène, 1,1,1,2,2-pentafluoropropane, et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf), et optionnellement ou non des impuretés ayant un point d'ébullition inférieur au point d'ébullition du 2,3,3,3-tetrafluoro-1-propène ;

ii') optionnellement ou non, distillation de ladite composition de 2,3,3,3-tetrafluoro-1-propène pour éliminer en tête de colonne des impuretés ayant un point d'ébullition inférieur au point d'ébullition du 2,3,3,3-tetrafluoro-1-propène et former un premier courant comprenant 2,3,3,3-tetrafluoro-1-propène, 1,1,1,2,2-pentafluoropropane, et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) récupéré en bas de colonne de distillation ;

iii') distillation dudit premier courant récupéré en bas de colonne de distillation à l'étape ii') ou de la composition **A** pour récupérer en tête de colonne de distillation un second courant comprenant du 2,3,3,3-tetrafluoro-1-propène et pour récupérer en bas de colonne de distillation un troisième courant comprenant 1,1,1,2,2-pentafluoropropane, et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) correspondant à ladite première composition mise en oeuvre à l'étape a) du procédé de purification du 1,1,1,2,2-pentafluoropropane selon la présente invention.

Lesdites impuretés ayant un point d'ébullition inférieur au point d'ébullition du 2,3,3,3-tetrafluoro-1-propène peuvent être trifluorométhane (F23), monofluorométhane (F41), difluorométhane (F32), pentafluoroéthane (F125), 1,1,1-trifluoroéthane (F143a), trifluoropropyne ou 1-chloro-pentafluoroéthane (F115).

Selon un second aspect, la présente invention fournit un procédé de production du 2,3,3,3-tetrafluoro-1-propène. En outre, ce procédé peut inclure la purification du 1,1,1,2,2-pentafluoropropane produit au cours de la production de 2,3,3,3-tetrafluoro-1-propène. Ainsi, la présente invention fournit un procédé de production de 2,3,3,3-tetrafluoro-1-propène comprenant les étapes de :

A) fluoration en présence d'un catalyseur d'un composé de formule $CX(Y)_2$-$CX(Y)_m$-$CH_m XY$ (I) dans laquelle X et Y représentent indépendamment H, F, ou Cl et m = 0 ou 1 ; et/ou fluoration en présence d'un catalyseur d'un composé de formule $(CX_nY_{3-n})CH_pX_{1-p}CH_mX_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant comprenant du 2,3,3,3-tetrafluoro-1-propene, du 1,1,1,2,2-pentafluoropropane, et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf);

C) distillation du courant récupéré à l'étape B) et récupération en tête de colonne de distillation un courant comprenant du 2,3,3,3-tetrafluoro-1-propene et en bas de colonne de distillation un courant comprenant du 1,1,1,2,2-pentafluoropropane et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf);

D) mise en oeuvre du procédé de purification 1,1,1,2,2-pentafluoropropane selon la présente invention à partir du courant récupéré en bas de colonne de distillation à l'étape C); et

E) recyclage à l'étape A) du courant comprenant du 1,1,1,2,2-pentafluoropropane formé à l'étape b) du procédé mis en oeuvre à l'étape D) ou déhydrofluoration du courant comprenant du 1,1,1,2,2-pentafluoropropane formé à l'étape b) du procédé de purification 1,1,1,2,2-pentafluoropropane mis en oeuvre à l'étape D) en l'absence ou en présence d'acide fluorhydrique.

Les réactions de l'étape A) peuvent être réalisées en présence d'acide fluorhydrique.

Le courant comprenant du 1,1,1,2,2-pentafluoropropane utilisé pour la mise en oeuvre de l'étape D) peut correspondre à ladite première composition décrite ci-dessus en relation avec le procédé de purification du 1,1,1,2,2-pentafluoropropane. Le courant comprenant du 1,1,1,2,2-pentafluoropropane obtenu en bas de colonne de distillation à l'étape C) et utilisé pour la mise en oeuvre de l'étape D) peut également comprendre au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf).

Selon un mode de réalisation préféré, le procédé de production de 2,3,3,3-tetrafluoro-1-propène comprenant les étapes de :

A) fluoration, en présence d'un catalyseur, d'un composé de formule $CX(Y)_2-CX(Y)_m-CH_mXY$ (I) dans laquelle X et Y représentent indépendamment H, F, ou Cl et m = 0 ou 1 ; et/ou fluoration en présence d'un catalyseur d'un composé de formule $(CX_nY_{3-n})CH_pX_{1-p}=CH_mX_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant comprenant du 2,3,3,3-tetrafluoro-1-propene, du 1,1,1,2,2-pentafluoropropane, et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf);

C) distillation du courant récupéré à l'étape B) et récupération en tête de colonne de distillation un courant comprenant du 2,3,3,3-tetrafluoro-1-propene et en bas de colonne de distillation un courant comprenant du 1,1,1,2,2-pentafluoropropane et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf);

D) mise en oeuvre du procédé de purification de 1,1,1,2,2-pentafluoropropane à partir du courant récupéré en bas de colonne de distillation à l'étape C), comprenant les étapes de :

a) mise en contact avec au moins un agent d'extraction organique du courant comprenant le 1,1,1,2,2-pentafluoropropane, trans-1,3,3,3-tetrafluoropropene et optionnellement au moins un des composés choisi parmi le groupe consistant en 1,1-difluoroéthane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf), pour former une seconde composition ;

b) Distillation extractive de ladite seconde composition pour former et récupérer :

i) une troisième composition comprenant ledit agent d'extraction organique, trans-1,3,3,3-tetrafluoropropene et optionnellement au moins un des composés choisi parmi le groupe consistant en 1,1-difluoroéthane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z) et 3,3,3-trifluoropropene (1243zf) ; et

ii) un courant comprenant le 1,1,1,2,2-pentafluoropropane,

c) récupération de ladite troisième composition et séparation entre ledit agent d'extraction organique et ledit au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z) et 3,3,3-trifluoropropene (1243zf),

E) recyclage à l'étape A) du courant comprenant du 1,1,1,2,2-pentafluoropropane formé et récupéré à l'étape b) du procédé de purification mis en oeuvre à l'étape D) ou déhydrofluoration du courant comprenant du 1,1,1,2,2-pentafluoropropane formé à l'étape b) du procédé de purification 1,1,1,2,2-pentafluoropropane mis en oeuvre à l'étape D) en l'absence ou en présence d'acide fluorhydrique.

Selon un mode de réalisation particulier, le courant récupéré à l'étape B) du procédé de production du 2,3,3,3-tétrafluoro-1-propène peut comprendre de l'acide fluorhydrique. Dans ce cas, préalablement à l'étape C), le courant récupéré à l'étape B) peut subir une distillation préalable B1') pour éliminer HF en bas de colonne de distillation. Un courant comprenant 2,3,3,3-tétrafluoro-1-propène, 1,1,1,2,2-pentafluoropropane, et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) est récupéré en tête de colonne de distillation. Ce dernier courant récupéré en tête de colonne de distillation est ensuite soumis à l'étape C) du procédé de production du 2,3,3,3-tetrafluoro-1-propène.

Selon un mode de réalisation particulier, le courant récupéré à l'étape B) du procédé de production du 2,3,3,3-tétrafluoro-1-propène peut également comprendre des impuretés ayant un point d'ébullition inférieur au point d'ébullition du 2,3,3,3-tetrafluoro-1-propène. Dans ce cas, préalablement à l'étape C), le courant récupéré à l'étape B) peut subir une distillation préalable B2'), subséquente à l'étape B1'), pour éliminer en tête de colonne de distillation lesdites impuretés ayant un point d'ébullition inférieur au point d'ébullition du 2,3,3,3-tetrafluoro-1-propène et former un premier courant comprenant 2,3,3,3-tetrafluoro-1-propène, 1,1,1,2,2-pentafluoropropane, et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) récupéré en bas de colonne de distillation. Ce dernier courant récupéré en bas de colonne de distillation est ensuite soumis à l'étape C) du procédé de production du 2,3,3,3-tetrafluoro-1-propène.

Selon un mode de réalisation particulier, le courant récupéré à l'étape B) du procédé de production du 2,3,3,3-tétrafluoro-1-propène peut également comprendre HCl. L'acide chlorhydrique peut être récupéré par distillation avant ou après l'étape B1' indépendamment des autres étapes du procédé.

Plus particulièrement, l'étape A) est effectuée à partir de 1,1,2,3-tetrachloropropène, le 2,3,3,3,-tetrachloropropène, le 1,1,3,3-tetrachloropropène, le 1,3,3,3-tetrachloropropène, le 1,1,1,2,3-pentachloropropane, le 1,1,1,3,3-pentachloropropane, le 1,1,2,2,3-pentachloropropane, le 1,2-dichloro-3,3,3-trifluoropropane, le 2-chloro-2,3,3,3-tetrafluoropropane, le 1,1,1,2,2-pentafluoropropane, le 1-chloro-1,3,3,3-tetrafluoropropane et le 1,1,1,3,3-pentafluoropropane, de préférence à partir de 1,1,1,2,3-pentachloropropane, 1,1,2,3,tetrachloropropène, du 1,1,1,2,2-pentafluoropropane et/ou du 2-chloro-3,3,3-trifluoro-1-propène ; en particulier à partir du 1,1,1,2,3-pentachloropropane (240db).

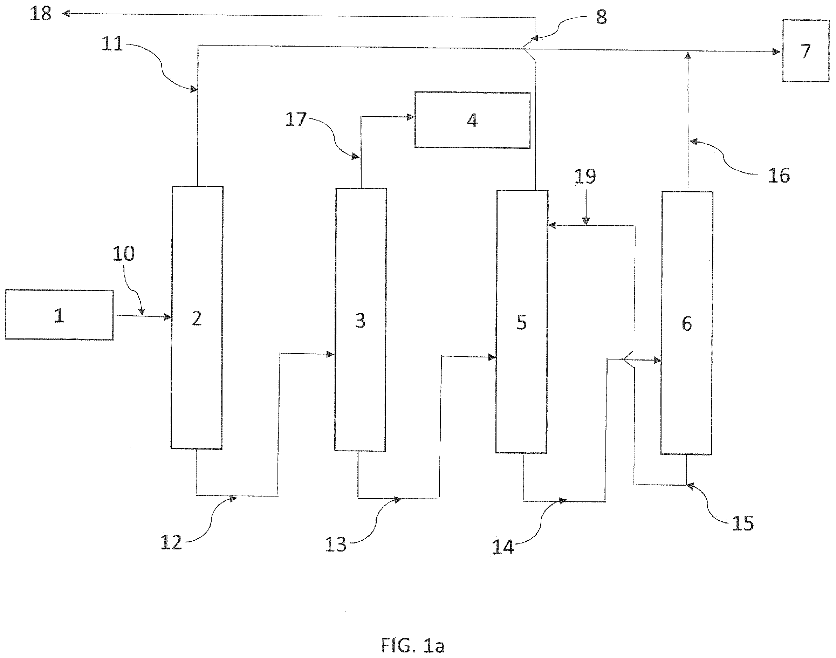

La figure 1a représente un schéma simplifié d'un dispositif mettant en oeuvre un procédé de purification du 1,1,1,2,2-pentafluoropropane.

Le mélange issu de la réaction de fluoration, en présence d'un catalyseur, d'un composé de formule $CX(Y)_2\text{-}CX(Y)_m\text{-}CH_mXY$ (I) dans laquelle X et Y représentent indépendamment H, F, ou CI et m = 0 ou 1 ; et/ou de fluoration en présence d'un catalyseur d'un composé de formule $(CX_nY_{3-n})CH_pX_{1-p}=CH_mX_{2-m}$ (II) est obtenu en 1. Le mélange comprend, dans ce mode de réalisation particulier, 2,3,3,3-tetrafluoro-1-propene, 1,1,1,2,2-pentafluoropropane, au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf), et des impuretés ayant un point d'ébullition inférieur à celui du 2,3,3,3-tetrafluoro-1-propène. Le mélange est transféré dans une colonne de distillation 2 par le conduit 10. Les impuretés ayant un point d'ébullition inférieur à celui du 2,3,3,3-tetrafluoro-1-propène sont récupérées en tête de la colonne de distillation 2 et acheminées par le conduit 11 à un incinérateur ou un dispositif de purification 7. Le résidu obtenu en bas de colonne de distillation et comprenant les autres constituants du mélange est acheminé vers une seconde colonne de distillation 3 par le conduit 12. La distillation effectuée en 3 vise à séparer le 2,3,3,3-tetrafluoro-1-propene des autres constituants du mélange. Les conditions opératoires de distillation sont donc adaptées à ce but. Le 2,3,3,3-tetrafluoro-1-propene est récupéré en tête de colonne de distillation et acheminé par le conduit 17 vers un dispositif de purification 4 apte à finaliser la purification du 2,3,3,3-tetrafluoropropène, par exemple une colonne de distillation. Le mélange récupéré en bas de la colonne de distillation 3 comprenant notamment le 1,1,1,2,2-pentafluoropropane et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf). Celui-ci est transféré par le conduit 13 dans un dispositif de distillation extractive 5 pour séparer le 1,1,1,2,2-pentafluoropropane d'au moins un des composés suivants 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf), en particulier pour le séparer du trans-1,3,3,3-tetrafluoro-1-propene. Le dispositif de distillation extractive 5 est alimenté par l'agent d'extraction organique 19 sélectionné selon la méthode décrite dans la présente demande. Le 1,1,1,2,2-pentafluoropropane est récupéré en tête du dispositif de distillation extractive 5 pour être acheminé par le conduit 8 vers un dispositif 18 de production du 2,3,3,3-tetrafluoro-1-propene. Le mélange récupéré en bas du dispositif de distillation extractive 5 comprend notamment l'agent d'extraction organique 19 et au moins un des composés suivants 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf), en particulier le trans-1,3,3,3-tetrafluoro-1-propene. Le mélange récupéré en bas du dispositif de distillation extractive 5 est acheminé par le conduit 14 vers un dispositif de distillation 6 visant à séparer l'agent d'extraction organique des autres composés présents. L'agent d'extraction organique est récupéré en bas du dispositif de distillation et recyclé par le conduit 15 vers le dispositif de distillation extractive 5. Les composés récupérés en tête du dispositif de distillation 6 sont acheminés par le conduit 16 vers le conduit 11 pour être incinérés ou purifiés en 7.

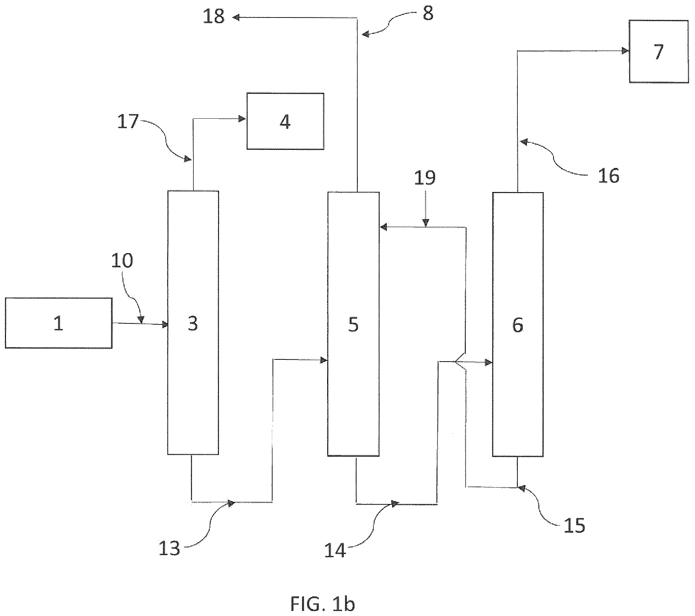

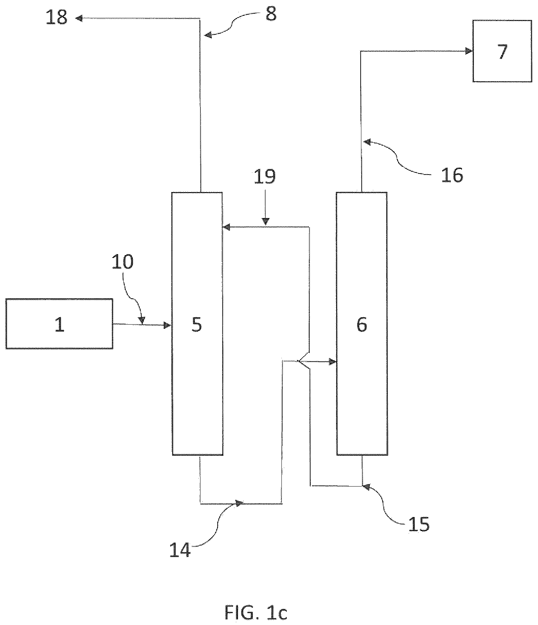

Le mélange fourni en 1 peut être dépourvu d'impuretés ayant un point d'ébullition inférieur à celui du 2,3,3,3-tetrafluoro-1-propène. Dans ce cas, comme illustré à la Fig. 1b, le mélange est acheminé par le conduit 10 vers la colonne de distillation 3 pour être traité comme expliqué ci-dessus en relation avec la Fig. 1a. Dans un autre mode de réalisation particulier illustré à la Fig. 1c, le mélange 1 peut comprendre le 1,1,1,2,2-pentafluoropropane et au moins un des composés suivants 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf). Dans ce cas, le mélange est acheminé directement vers la colonne de distillation extractive 5 pour y être traité comme expliqué ci-dessus en relation avec la Fig. 1a.

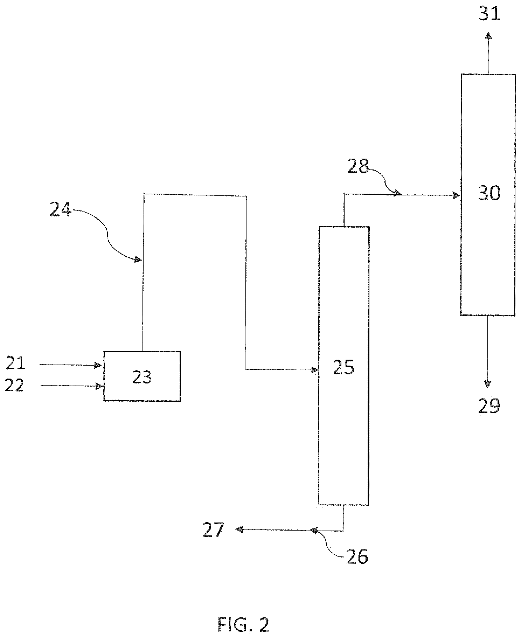

La Fig. 2 illustre schématiquement un dispositif mettant en oeuvre un procédé de production du 2,3,3,3-tetrafluoropropène.

L'acide fluorhydrique 21 est mis en contact avec du 1,1,1,2,3-pentachloropropane (240db) 22 dans un réacteur 23. Le mélange obtenu et comprenant 2,3,3,3-tetrafluoropropène, 1,1,1,2,2-pentafluoropropane (245cb) et au moins un des composés suivants 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) est récupéré en sortie de réacteur et acheminé vers une colonne de distillation 25 par le conduit 24. Le mélange peut aussi comprendre HCl, HF et des impuretés lourdes ou ayant un point d'ébullition inférieur à celui du 2,3,3,3-tetrafluoro-1-propène. Le courant obtenu en bas de colonne de distillation comprenant HF et éventuellement des impuretés lourdes est acheminé vers le dispositif de purification 27 via le conduit 26 pour purifié HF qui sera recyclé éventuellement en 23. Les autres constituants du mélange sont acheminés via le conduit 28 vers une colonne de distillation 30. Le courant 31 obtenu en tête de colonne de distillation contient du HCl et le courant obtenu en bas de colonne de distillation est acheminé vers un dispositif de purification 29 de purification du 1,1,1,2,2-pentafluoropropane. Le dispositif de purification 29 peut être l'un quelconque des dispositifs illustrés aux Fig. 1a-1b.

Le catalyseur utilisé dans le présent procédé de production de 2,3,3,3-tétrafluoropropène peut être par exemple à base d'un métal comprenant un oxyde de métal de transition ou un dérivé ou un halogénure ou un oxyhalogénure d'un tel métal. On peut citer par exemple $FeCl_3$, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple $AlF_3$ et $Al_2O_3$, l'oxyfluorure d'alumine et le fluorure d'alumine).

On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb.

On peut faire référence à cet égard au document WO 2007/079431 (en p.7, l.1-5 et 28-32), au document EP 939071 (paragraphe [0022]), au document WO 2008/054781 (en p.9 l.22-p.10 l.34), et au document WO 2008/040969 (revendication 1), auxquels il est fait expressément référence.

Le catalyseur est de manière plus particulièrement préférée à base de chrome et il s'agit plus particulièrement d'un catalyseur mixte comprenant du chrome.

Selon un mode de réalisation, on utilise un catalyseur mixte comprenant du chrome et du nickel. Le rapport molaire Cr / Ni (sur la base de l'élément métallique) est généralement de 0,5 à 5, par exemple de 0,7 à 2, par exemple d'environ 1. Le catalyseur peut contenir de 0,5 à 20 % en poids de nickel.

Le métal peut être présent sous forme métallique ou sous forme de dérivé, par exemple un oxyde, halogénure ou oxyhalogénure. Ces dérivés sont de préférence obtenus par activation du métal catalytique.

Le support est de préférence constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838, ou obtenus par le procédé d'activation décrit ci-dessus.

Le catalyseur peut comprendre du chrome et du nickel sous une forme activée ou non, sur un support qui a été soumis à une activation ou non.

On peut se reporter au document WO 2009/118628 (notamment en p.4, l.30-p.7 l.16), auquel il est fait expressément référence ici.

Un autre mode de réalisation préféré repose sur un catalyseur mixte contenant du chrome et au moins un élément choisi parmi Mg et Zn. Le rapport atomique de Mg ou Zn/Cr est de préférence de 0,01 à 5.

Avant son utilisation, le catalyseur est de préférence soumis à une activation avec de l'air, de l'oxygène ou du chlore et/ou avec de l'HF.

Par exemple, le catalyseur est de préférence soumis à une activation avec de l'air ou de l'oxygène et du HF à une température de 100 à 500°C, de préférence de 250 à 500°C et plus particulièrement de 300 à 400°C. La durée d'activation est de préférence de 1 à 200 h et plus particulièrement de 1 à 50 h.

Cette activation peut être suivie d'une étape d'activation de fluoration finale en présence d'un agent d'oxydation, d'HF et de composés organiques.

Le rapport molaire HF / composés organiques est de préférence de 2 à 40 et le rapport molaire agent d'oxydation / composés organiques est de préférence de 0,04 à 25. La température de l'activation finale est de préférence de 300 à 400°C et sa durée de préférence de 6 à 100 h.

La réaction de fluoration en phase gazeuse peut être effectuée :

- avec un rapport molaire HF / composé de formule (I) et/ou (II) de 3:1 à 150:1, de préférence de 4:1 à 125:1 et de manière plus particulièrement préférée de 5:1 à 100:1 ;
- avec un temps de contact de 3 à 100 s, de préférence 4 à 75 s et plus particulièrement 5 à 50 s (volume de catalyseur divisé par le flux entrant total, ajusté à la température et à la pression de fonctionnement) ;
- à une pression allant de la pression atmosphérique à 20 bar, de préférence de 2 à 18 bar et plus particulièrement de 3 à 15 bars;
- à une température (température du lit de catalyseur) de 200 à 450°C, de préférence de 250 à 400°C, et plus particulièrement de 280 à 380°C.

La durée de l'étape de réaction est typiquement de 10 à 8000 heures, de préférence de 50 à 5000 heures et de manière plus particulièrement préférée de 70 à 1000 heures.

Un agent oxydant, de préférence l'oxygène, peut éventuellement être ajouté lors de la réaction de fluoration. Le rapport molaire oxygène / composés organiques peut être de 0,005 à 2, de préférence de 0,01 à 1,5. L'oxygène peut être introduit pur ou sous forme d'air ou de mélange oxygène / azote. On peut également remplacer l'oxygène par du chlore.

Une composition azéotropique ou quasi-azéotropique est décrite.

Ladite composition comprend de 50 à 99,99% en poids de 1,1,1,2,2-pentafluoropropane, moins de 50 % en poids de trans-1,3,3,3-tetrafluoro-1-propène sur base du poids total de la composition. De préférence, ladite composition comprend également au moins un des composés sélectionnés parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf). Ledit au moins un des composés sélectionnés parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) peut être présent dans la composition azéotropique dans les proportions exprimées ci-dessus sur base du poids total de la composition. Ladite composition azéotropique ou quasi-azéotropique peut être obtenue à une température de -30°C à 50°C. Ladite composition azéotropique ou quasi-azéotropique peut être obtenue pour une pression de 0,5 bar à 10 bars.

**Méthode de sélection de l'agent d'extraction organique**

La sélection de l'agent d'extraction organique est déterminée par l'utilisation du modèle Cosmo-RS implémenté dans le logiciel COSMOTHERM. Pour ce couple binaire sélectionné, un facteur de séparation est calculé pour chacun des solvants étudiés par l'équation suivante :

$$S_{1,2} = (\gamma_{1,S} * P1)/(\gamma_{2,S} * P2)$$

dans laquelle

$\gamma_{1,S}$ représente le coefficient d'activité du premier composé 1 dans l'agent d'extraction organique considéré à dilution infinie,
P1 représente la pression de vapeur saturante du premier composé 1,
$\gamma_{2,S}$ représente le coefficient d'activité du second composé 2 du couple binaire dans l'agent d'extraction organique considéré à dilution infinie,
P2 représente la pression de vapeur saturante du second composé.

Une capacité d'absorption est également calculée pour chacun des solvants étudiés et pour un couple binaire (1,2) considéré. La capacité d'absorption est calculée par la formule $C_{2,S} = 1/(\gamma_{2,S})$ dans laquelle $\gamma_{2,S}$ représente le coefficient d'activité du second composé du couple binaire considéré dans ledit agent d'extraction organique étudié à dilution infinie.
Les calculs sont répétés pour chaque agent d'extraction organique étudié. Des valeurs minimales de facteur de séparation et de capacité d'absorption sont identifiées afin de permettre une séparation suffisante entre le premier composé et le second composé du couple binaire (1,2) considéré. La pression de vapeur saturante est considérée pour une température de 25°C.

**Exemples**

Exemple 1

Dans cet exemple, la séparation entre le 1,1,1,2,2-pentafluoropropane (245cb) et le trans-1,3,3,3-tetrafluoro-1-propene est considéré. Sur base des informations obtenues par le modèle Cosmo-RS, les solvants repris dans le tableau 1 ci-dessous ont été testés pour la distillation extractive d'un mélange comprenant 1,1,1,2,2-pentafluoropropane (245cb) et le trans-1,3,3,3-tetrafluoro-1-propene.

Tableau 1- Capacité et Facteur de séparation de l'agent d'extraction organique

| Agent extraction organique | Capacité d'absorption | Facteur de séparation |
|---|---|---|
| éthylamine | 1,95 | 3,60 |
| isopropylamine | 1,85 | 3,14 |
| n-propylamine | 1,86 | 3,14 |
| diéthylamine | 1,57 | 2,18 |
| tétrahydrofurane | 1,73 | 1,90 |
| éthylacétate | 1,30 | 1,87 |
| butanone | 1,07 | 1,84 |
| 3-pentylamine | 1,62 | 2,39 |
| 2-methoxyethanamine | 1,98 | 3,80 |
| Dioxane | 1,09 | 1,92 |
| n-pentylamine | 1,64 | 2,71 |
| 1,3-dioxane | 1,09 | 1,93 |
| 1,2-diaminoethane | 1,28 | 6,88 |

(suite)

| Agent extraction organique | Capacité d'absorption | Facteur de séparation |
|---|---|---|
| 2-methoxyethanol | 0,57 | 2,05 |
| 1,2-propanediamine | 1,50 | 4,92 |
| n-butylacetate | 1,35 | 1,86 |
| 1-ethoxy-2-propanol | 1,12 | 2,15 |

Les résultats ont été confirmés à partir d'un mélange comprenant 70 à 90% en poids de 1,1,1,2,2-pentafluoropropane et 10 à 30 % en poids de trans-1,3,3,3-tetrafluoro-1-propene sur base du poids total de la composition. Le reste de la composition est formée par l'agent d'extraction organique testé.

**Revendications**

1. Procédé de purification du 1,1,1,2,2-pentafluoropropane (245cb) à partir d'une première composition comprenant du 1,1,1,2,2-pentafluoropropane et trans-1,3,3,3-tetrafluoro-1-propene (1234ze-E) et optionnellement au moins un des composés choisi parmi le groupe consistant en 1,1-difluoroéthane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf), ledit procédé comprenant les étapes de:

   a) mise en contact de ladite première composition avec au moins un agent d'extraction organique pour former une seconde composition ;
   b) distillation extractive de ladite seconde composition pour former :

   i) une troisième composition comprenant ledit agent d'extraction organique, trans-1,3,3,3-tetrafluoro-1-propene (1234ze-E) et optionnellement ledit au moins un des composés choisi parmi le groupe consistant en 1,1-difluoroéthane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z) et 3,3,3-trifluoropropene (1243zf) ; et
   ii) un courant comprenant le 1,1,1,2,2-pentafluoropropane,

   c) récupération et séparation de ladite troisième composition pour former un courant comprenant ledit agent d'extraction organique et un courant comprenant trans-1,3,3,3-tetrafluoro-1-propene (1234ze-E), et optionnellement ledit au moins un des composés choisi parmi le groupe consistant en 1,1-difluoroéthane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z) et 3,3,3-trifluoropropene (1243zf), de préférence, l'agent d'extraction organique séparé à l'étape c) est recyclé à l'étape a) ; et **caractérisé en ce que** ledit agent d'extraction organique est sélectionné parmi le groupe consistant en éthylamine, bromofluoromethane, 1-bromo-1,2-difluoroethylene, acetaldehyde, 1,1,1-trifluoro-2-propanone, 1,1,1-trifluoro-2-bromoethane, 2,2,2-trifluoroethylmethylether, isopropylamine, methylformate, 2-methoxy-1-propene, diethylether, 1,2-epoxypropane, ethanethiol, ethoxy-ethene, ethylmethylamine, dimethylsulfide, 2-chloropropane, bromoethane, dimethoxymethane, iodomethane, 2-amino-2-methylpropane, methylcyclopropylether, n-propylamine, isopropylmethylamine, ethanedial, 2-chloro-2-methylpropane, 2-propanethiol, 2-ethoxy-propane, methyl-t-butylether, diethylamine, propanone, methylacetate, 4-methoxy-2-methyl-2-butanethiol, 2-bromopropane, chloromethoxymethane, 2-butanamine, n-methylpropylamine, tert-butylthiol, isobutanal, methanol, tetrahydrofurane, 1-propanethiol, chlorobromomethane, isopropylformate, diisopropylether, 3-bromopropene, 1-bromopropane, methylglyoxal, iodoethane, 2-ethoxy-2-methyl-propane, 2-bromo-2-methylpropane, 2,2,2-trifluoroethanol, 1-chloro-3-fluoropropane, 1,1,1-trifluoro-2-propanol, 1-butylamine, ethylacetate, ethanol, butanone, n-propylformate, 2-ethoxy-butane, 2-propanol, acetonitrile, tert-butanol, 1-methoxy-2-methyl-butane, 2,2-dimethoxypropane, 2-chloro-2-methylbutane, 1,2-dichloroethane, 1-ethoxy-2-methylpropane, diisopropylamine, 2-butanethiol, 1,2-dimethoxyethane, 3-methyl-2-butanamine, diethoxymethane, 2-methyl-1-propanethiol, isopropylacetate, 2-iodopropane, di-n-propylether, 3-pentylamine, n-methylbutylamine, 2-bromobutane, diethylsulfide, 1-ethoxybutane, 1-methoxy-2-propanamine, 2-methylbutanal, 2-methoxyethanamine, 2,2-difluoroethanol, 1,2-dichloropropane, propanol, tert-butylacetate, propionitrile, trichloroacetaldehyde, 2-allyloxyethanol, butanethiol, 1-methoxy-pentane, ethylpropionate, 2-butanol, 1,2-dimethoxypropane, isopropyl-isobutyl-ether, 1-chloro-4-fluorobutane, 1-bromo-3-fluoropropane, dioxane, 1-bromobutane, 3-pentanone, 1,1-diethoxyethane, 2-pentanone, 2-methyl-2-butanol, 1-iodopropane, 2-methoxy-1propanamine, trimethoxymethane, cis-1,3-dichloropropene, n-pentylamine, 3,3-dimethyl-2-butanone, 1,3-dioxane, piperidine, 1-bromo-2-chloroethane, isobutanol, 2-bromo-2-methylbutane, dipropylamine, 2,2,3,3-tetraflouro-1-propanol, 2-ethoxyethanamine, triethylfluorosilane, sec-butylacetate, trans-1,3-dichloropropene, 2,2-dimethyl-1-propanol, n-methyl-1,2-ethanediamine, 2,2-diethoxypropane, 1,3,5-trioxane, pyridine, n-methylmorpholine, 3-pentanol, 4-methyl-2-pentanone, 1,2-diaminoethane, isobutyl-tert-butylether, 2-bromopentane, butyronitrile, 1-butanol, 2,3-dichlorobutane, sec-butyl-tert-butylether, 1-methoxy2-propanol, 1,2-propanediamine, 2,6-dimethyl-5-heptenal, 1-bromo-3-methylbutane, 1,3-dichloro-trans-2-butene, 1,3-dichloropropane, 1-(dimethylamino)-2-propanol, tetrahydrothiophene, 3-methyl-3-pentanol, 1,2-dibromo-1-fluoroethane, 1,1-diethoxypropane, 1,2,2-trichloropropane, 1-chloro-2-methyl-2-propanol, 2-methoxyethanol, 4,4,4-trifluorobutanol, 2-ethylbutylamine, diethylcarbonate, n-butylacetate, 1-pentanethiol, 2-chloro-1,1-dimethoxyethane, 2-hexanone, n-ethylethylenediamine, 3-fluoropropanol, 5-hexen-2-one, 2,3-dichloro-2-methylbutane, 1,1-diethoxy-n,n-dimethylmethanamine, 2-methylpyridine, 1-bromopentane, 2-methoxy1-propanol, 1,2-dichloro-2-butene, 1-iodobutane, hexanal, 1-ethoxy-2-propanol, 1,2-dibromoethane, 4-methyl-2-pentanol, bromoaceticacidmethylester, 1,1,2-trichloropropane, 1,2-octanediol, 4-methyl-2-hexamine, hexylamine, 2-chloro-1-propanol, methoxycyclohexane, 2-(dimethylamino)-ethanol, 1,3-dichlorobutane, cyclohexylamine, n-ethyl-2-dimethylaminoethylamine, ethoxyethanol, 3-hexanol, 2-hexanol, 2-methylpyrazine, 2-ethoxy-1-propanol, 1-pentanol, n-ethyl-morpholine, 1-methylpiperazine, 1,3-propanediamine, di-n-butylether, valeronitrile, (methyleneamino)acetonitrile, 1,2-dibromopropane, 1,2,3-trichloropropene, 2-heptanamine, 2,3-dimethylbutanol, 1-ethoxy-hexane, 1-chloro-3-bromopropane, n,n-diethylethylenediamine, 3-furfural, 2,6-dimethylpyridine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, 2-ethyl-1-butanol, 2-methyl-1-pentanol, methylhexanoate, 2-propoxyethanol, 1-propoxy-2-propanol, dimethylethanolamine.

2. Procédé selon la revendication 1 **caractérisé en ce que** la première composition est une composition azéotropique ou quasi-azéotropique comprenant 1,1,1,2,2-pentafluoropropane (245cb) et trans-1,3,3,3-tetrafluoro-1-propene (1234ze-E) et obtenue à une température de -30°C à 50°C à une pression de 0,5 à 10 bars.

3. Procédé selon l'une quelconque des revendications précédentes **caractérisé en ce que** ledit agent d'extraction organique est choisi parmi le groupe consistant en éthylamine, isopropylamine, n-propylamine, diethylamine, tetrahydrofurane, ethylacetate, butanone, 3-pentylamine, 2-methoxyethanamine, dioxane, 2-pentanone, n-pentylamine, 1,3-dioxane, 1,2-diaminoethane, 1,2-propanediamine, 2-methoxyethanol, n-butylacetate, 1-ethoxy-2-propanol.

4. Procédé selon l'une quelconque des revendications précédentes **caractérisé en ce que** ledit courant comprenant le 1,1,1,2,2-pentafluoropropane formé à l'étape b) est récupéré pour être utilisé dans un procédé de production du 2,3,3,3-tetrafluoropropène.

5. Procédé de production de 2,3,3,3-tetrafluoro-1-propène comprenant les étapes de :

A) fluoration en présence d'un catalyseur d'un composé de formule (I) CX(Y)$_2$-CX(Y)$_m$-CH$_m$XY dans laquelle X et Y représentent indépendamment un atome d'hydrogène, de fluor ou de chlore et m = 0 ou 1 ; et/ou fluoration en présence d'un catalyseur d'un composé de formule (CX$_n$Y$_{3-n}$)CH$_p$X$_{1-p}$CH$_m$X$_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant comprenant du 2,3,3,3-tetrafluoro-1-propene, du 1,1,1,2,2-pentafluoropropane, et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf) ;

C) distillation du courant récupéré à l'étape B) et récupération en tête de colonne de distillation d'un courant comprenant du 2,3,3,3-tetrafluoro-1-propene et en bas de colonne de distillation un courant comprenant du 1,1,1,2,2-pentafluoropropane et au moins un des composés choisi parmi le groupe consistant en 1,1-difluoroéthane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z), 3,3,3-trifluoropropene (1243zf);

D) mise en oeuvre du procédé de purification du 1,1,1,2,2-pentafluoropropane selon l'une quelconque des revendications 1 à 4 à partir du courant récupéré en bas de colonne de distillation à l'étape C); et

E) recyclage à l'étape A) du courant comprenant du 1,1,1,2,2-pentafluoropropane formé et récupéré à l'étape b) du procédé de purification mis en oeuvre à l'étape D) ou déhydrofluoration du courant comprenant du 1,1,1,2,2-pentafluoropropane formé à l'étape b) du procédé de purification 1,1,1,2,2-pentafluoropropane mis en oeuvre à l'étape D) en l'absence ou présence d'acide fluorhydrique.

**6.** Composition comprenant du 1,1,1,2,2-pentafluoropropane, trans-1,3,3,3-tetrafluoro-1-propene et un agent d'extraction organique sélectionné parmi le groupe consistant en éthylamine, isopropylamine, n-propylamine, diéthylamine, tetrahydrofurane, éthylacétate, butanone, 3-pentylamine, 2-methoxyethanamine, dioxane, 2-pentanone, n-pentylamine, 1,3-dioxane, 1,2-diaminoethane, 1,2-propanediamine, 2-methoxyethanol, n-butylacetate, 1-ethoxy-2-propanol.

**Patentansprüche**

**1.** Verfahren zur Reinigung von 1,1,1,2,2-Pentafluorpropan (245cb) ausgehend von einer ersten Zusammensetzung, die 1,1,1,2,2-Pentafluorpropan und trans-1,3,3,3-Tetrafluor-1-propen (1234ze-E) und gegebenenfalls mindestens eine Verbindung aus der Gruppe bestehend aus 1,1-Difluorethan (152a), 1,1,1,2-Tetrafluorethan (134a), cis-1,3,3,3-Tetrafluor-1-propen (1234ze-Z), trans-1,2,3,3,3-Pentafluorpropen (1225ye-E), cis-1,2,3,3,3-Pentafluorpropen (1225ye-Z) und 3,3,3-Trifluorpropen (1243zf) umfasst, wobei das Verfahren folgende Schritte umfasst:

a) Inkontaktbringen der ersten Zusammensetzung mit mindestens einem organischen Extraktionsmittel zur Bildung einer zweiten Zusammensetzung;
b) Extraktivdestillation der zweiten Zusammensetzung zur Bildung:

i) einer dritten Zusammensetzung, die das organische Extraktionsmittel und trans-1,3,3,3-Tetrafluor-1-propen (1234ze-E) und gegebenenfalls die mindestens eine Verbindung aus der Gruppe bestehend aus 1,1-Difluorethan (152a), 1,1,1,2-Tetrafluorethan (134a), cis-1,3,3,3-Tetrafluor-1-propen (1234ze-Z), trans-1,2,3,3,3-Pentafluorpropen (1225ye-E), cis-1,2,3,3,3-Pentafluorpropen (1225ye-Z) und 3,3,3-Trifluorpropen (1243zf) umfasst; und
ii) eines Stroms, der 1,1,1,2,2-Pentafluorpropan umfasst;

c) Gewinnung und Trennung der dritten Zusammensetzung zur Bildung eines Stroms, der das organische Extraktionsmittel umfasst, und eines Stroms, der trans-1,3,3,3-Tetrafluor-1-propen (1234ze-E) und gegebenenfalls die mindestens eine der Verbindungen aus der Gruppe bestehend aus 1,1-Difluorethan (152a), 1,1,1,2-Tetrafluorethan (134a), cis-1,3,3,3-Tetrafluor-1-propen (1234ze-Z), trans-1,2,3,3,3-Pentafluorpropen (1225ye-E), cis-1,2,3,3,3-Pentafluorpropen (1225ye-Z) und 3,3,3-Trifluorpropen (1243zf) umfasst; wobei das in Schritt c) abgetrennte organische Extraktionsmittel vorzugsweise zu Schritt a) zurückgeführt wird; und **dadurch gekennzeichnet, dass** das organische Extraktionsmittel aus der Gruppe bestehend aus Ethylamin, Bromfluormethan, 1-Brom-1,2-difluorethylen, Acetaldehyd, 1,1,1-Trifluor-2-propanon, 1,1,1-Trifluor-2-bromethan, 2,2,2-Trifluorethylmethylether, Isopropylamin, Methylformiat, 2-Methoxy-1-propen, Diethylether, 1,2-Epoxypropan, Ethanthiol, Ethoxyethen, Ethylmethylamin, Dimethylsulfid, 2-Chlorpropan, Bromethan, Dimethoxymethan, Iodmethan, 2-Amino-2-methylpropan, Methylcyclopropylether, n-Propylamin, Isopropylmethylamin, Ethandial, 2-Chlor-2-methylpropan, 2-Propanthiol, 2-Ethoxypropan, Methyl-t-butylether, Diethylamin, Propanon, Methylacetat, 4-Methoxy-2-methyl-2-butanthiol, 2-Brompropan, Chlormethoxymethan, 2-Butanamin, n-Methylpropylamin, tert-Butylthiol, Isobutanal, Methanol, Tetrahydrofuran, 1-Propanthiol, Chlorbrommethan, Isopropylformiat, Diisopropylether, 3-Brompropen, 1-Brompropan, Methylglyoxal, Iodethan, 2-Ethoxy-2-methylpropan, 2-Brom-2-methylpropan, 2,2,2-Trifluorethanol, 1-Chlor-3-fluorpropan, 1,1,1-Trifluor-2-propanol, 1-Butylamin, Ethylacetat, Ethanol, Butanon, n-Propylformiat, 2-Ethoxybutan, 2-Propanol, Acetonitril, tert-Butanol, 1-Methoxy-2-methylbutan, 2,2-Dimethoxypropan, 2-Chlor-2-methylbutan, 1,2-Dichlorethan, 1-Ethoxy-2-methylpropan, Diisopropylamin, 2-Butanthiol, 1,2-Dimethoxyethan, 3-Methyl-2-butanamin, Diethoxymethan, 2-Methyl-1-propanthiol, Isopropylacetat, 2-Iodpropan, Di-n-propylether, 3-Pentylamin, n-Methylbutylamin, 2-Brombutan, Diethylsulfid, 1-Ethoxybutan, 1-Methoxy-2-propanamin, 2-Methylbutanal, 2-Methoxyethanamin, 2,2-Difluorethanol, 1,2-Dichlorpropan, Propanol, tert-Butylacetat, Propionitril, Trichloracetaldehyd, 2-Allyloxyethanol, Butanthiol, 1-Methoxypentan, Ethylpropionat, 2-Butanol, 1,2-Dimethoxypropan, Isopropylisobutylether, 1-Chlor-4-fluorbutan, 1-Brom-3-fluorpropan, Dioxan, 1-Brombutan, 3-Pentanon, 1,1-Diethoxyethan, 2-Pentanon, 2-Methyl-2-butanol, 1-Iodpropan, 2-Methoxy-1-propanamin, Trimethoxymethan, cis-1,3-Dichlorpropen, n-Pentylamin, 3,3-Dimethyl-2-butanon, 1,3-Dioxan, Piperidin, 1-Brom-2-chlorethan, Isobutanol, 2-Brom-2-methylbutan, Dipropylamin, 2,2,3,3-Tetrafluor-1-propanol, 2-Ethoxyethanamin, Triethylfluorsilan, sec-Butylacetat, trans-1,3-Dichlorpropen, 2,2-Dimethyl-1-propanol, n-Methyl-1,2-ethan-diamin, 2,2-Diethoxypropan, 1,3,5-Trioxan, Pyridin, n-Methylmorpholin, 3-Pentanol, 4-Methyl-2-pentanon, 1,2-Diaminoethan, Isobutyl-tert-butylether, 2-Brompentan, Butyronitril, 1-Butanol, 2,3-Dichlorbutan, sec-Butyl-tert-butylether, 1-Methoxy-2-propanol, 1,2-Propandiamin, 2,6-Dimethyl-5-heptenal, 1-Brom-3-methylbutan, 1,3-Dichlor-trans-2-buten, 1,3-Dichlorpropan, 1-(Dimethylamino)-2-propanol, Tetrahydrothiophen, 3-Methyl-3-pentanol, 1,2-Dibrom-1-fluorethan, 1,1-Diethoxypropan, 1,2,2-Trichlorpropan, 1-Chlor-2-methyl-2-propanol, 2-Methoxyethanol, 4,4,4-Trifluorbutanol, 2-Ethylbutylamin, Diethylcarbonat, n-Butylacetat, 1-Pentanthiol, 2-Chlor-1,1-dimethoxyethan, 2-Hexanon, n-Ethylethylendiamin, 3-Fluorpropanol, 5-Hexen-2-on, 2,3-Dichlor-2-methylbutan, 1,1-Diethoxy-n,n-dimethylmethanamin, 2-Methylpyridin, 1-Brompentan, 2-Methoxy-1-propanol, 1,2-Dichlor-2-buten, 1-Iodbutan, Hexanal, 1-Ethoxy-2-propanol, 1,2-Dibromethan, 4-Methyl-2-pentanol, Bromessigsäuremethylester, 1,1,2-Trichlorpropan, 1,2-Octandiol, 4-Methyl-2-hexanamin, Hexylamin, 2-Chlor-1-propanol, Methoxycyclohexan, 2-(Dimethylamino)ethanol, 1,3-Dichlorbutan, Cyclohexylamin, n-Ethyl-2-dimethylaminoethylamin, Ethoxyethanol, 3-Hexanol, 2-Hexanol, 2-Methylpyrazin, 2-Ethoxy-1-propanol, 1-Pentanol, n-Ethylmorpholin, 1-Methylpiperazin, 1,3-Propandiamin, Di-n-butylether, Valeronitril, (Methylenamino)acetonitril, 1,2-Dibrompropan, 1,2,3-Trichlorpropen, 2-Heptanamin, 2,3-Dimethylbutanol, 1-Ethoxyhexan, 1-Chlor-3-brompropan, n,n-Diethylethylendiamin, 3-Furfural, 2,6-Dimethylpyridin, 4-Methyl-2-hexanon, 1,1,1-Triethoxyethan, 1-Methoxy-2-acetoxypropan, 4-Methylpyridin, n,n'-Diethyl-1,2-ethandiamin, 2,6-Dimethylmorpholin, 2-Ethyl-1-butanol, 2-Methyl-1-pentanol, Methylhexanoat, 2-Propoxyethanol, 1-Propoxy-2-propanol und Dimethylethanolamin ausgewählt wird.

2. Verfahren nach Anspruch 1, **dadurch gekennzeichnet, dass** es sich bei der ersten Zusammensetzung um eine azeotrope oder quasiazeotrope Zusammensetzung handelt, die 1,1,1,2,2-Pentafluorpropan (245cb) und trans-1,3,3,3-Tetrafluor-1-propen (1234ze-E) umfasst und bei einer Temperatur von -30 °C bis 50 °C und bei einem Druck von 0,5 bis 10 bar erhalten wird.

3. Verfahren nach einem der vorhergehenden Ansprüche, **dadurch gekennzeichnet, dass** das organische Extraktionsmittel aus der Gruppe bestehend aus Ethylamin, Isopropylamin, n-Propylamin, Diethylamin, Tetrahydrofuran, Ethylacetat, Butanon, 3-Pentylamin, 2-Methoxyethanamin, Dioxan, 2-Pentanon, n-Pentylamin, 1,3-Dioxan, 1,2-Diaminoethan, 1,2-Propandiamin, 2-Methoxyethanol, n-Butylacetat und 1-Ethoxy-2-propanol ausgewählt wird.

4. Verfahren nach einem der vorhergehenden Ansprüche, **dadurch gekennzeichnet, dass** der in Schritt b) gebildete Strom, der 1,1,1,2,2-Pentafluorpropan umfasst, zur Verwendung bei einem Verfahren zur Herstellung von 2,3,3,3-Tetrafluorpropen zurückgewonnen wird.

5. Verfahren zur Herstellung von 2,3,3,3-Tetrafluor-1-propen, das folgende Schritte umfasst:

A) Fluorierung einer Verbindung der Formel (I) $CX(Y)_2$-$CX(Y)_m$-$CH_mXY$, in der X und Y unabhängig für ein Wasserstoff-, Fluor- oder Chloratom stehen und m = 0 oder 1, in Gegenwart eines Katalysators und/oder Fluorierung einer Verbindung der Formel $(CX_nY_{3-n})CH_pX_{1-p}CH_mX_{2-m}$ (II), in der X unabhängig voneinander für Cl, F, I oder Br steht; Y unabhängig voneinander für H, Cl, F, I oder Br steht; n für 1, 2 oder 3 steht und m für 0, 1 oder 2 steht und p für 0 oder 1 steht, in Gegenwart eines Katalysators;

B) Rückgewinnung eines Stroms, der 2,3,3,3-Tetrafluor-1-propen, 1,1,1,2,2-Pentafluorpropan und mindestens eine Verbindung aus der Gruppe bestehend aus 1,1-Difluorethan (152a), 1,1,1,2-Tetrafluorethan (134a), trans-1,3,3,3-Tetrafluor-1-propen (1234ze-E), cis-1,3,3,3-Tetrafluor-1-propen (1234ze-Z), trans-1,2,3,3,3-Pentafluorpropen (1225ye-E), cis-1,2,3,3,3-Pentafluorpropen (1225ye-Z) und 3,3,3-Trifluorpropen (1243zf) umfasst;

C) Destillation des in Schritt B) gewonnenen Stroms und Rückgewinnung eines Stroms, der 2,3,3,3-Tetrafluor-1-propen umfasst, am Kopf der Destillationssäule und eines Stroms, der 1,1,1,2,2-Pentafluorpropan und mindestens eine Verbindung aus der Gruppe bestehend aus 1,1-Difluorethan (152a), 1,1,1,2-Tetrafluorethan (134a), trans-1,3,3,3-Tetrafluor-1-propen (1234ze-E), cis-1,3,3,3-Tetrafluor-1-propen (1234ze-Z), trans-1,2,3,3,3-Pentafluorpropen (1225ye-E), cis-1,2,3,3,3-Pentafluorpropen (1225ye-Z) und 3,3,3-Trifluorpropen (1243zf) umfasst, am Sumpf der Destillationssäule;

D) Durchführung des Verfahrens zur Reinigung von 1,1,1,2,2-Pentafluorpropan nach einem der Ansprüche 1 bis 4 unter Verwendung des in Schritt C) am Sumpf der Destillationssäule gewonnenen Stroms und

E) Zurückführung des in Schritt b) des in Schritt D) durchgeführten Reinigungsverfahrens gebildeten und zurückgewonnenen Stroms, der 1,1,1,2,2-Pentafluorpropan umfasst, zu Schritt A) oder Dehydrofluorierung des in Schritt b) des in Schritt D) durchgeführten Verfahrens zur Reinigung von 1,1,1,2,2-Pentafluorpropan gebildeten Stroms, der 1,1,1,2,2-Pentafluorpropan umfasst, in Abwesenheit oder Anwesenheit von Fluorwasserstoffsäure.

6. Zusammensetzung, umfassend 1,1,1,2,2-Pentafluorpropan und trans-1,3,3,3-Tetrafluor-1-propen und ein organisches Extraktionsmittel aus der Gruppe bestehend aus Ethylamin, Isopropylamin, n-Propylamin, Diethylamin, Tetrahydrofuran, Ethylacetat, Butanon, 3-Pentylamin, 2-Methoxyethanamin, Dioxan, 2-Pentanon, n-Pentylamin, 1,3-Dioxan, 1,2-Diaminoethan, 1,2-Propandiamin, 2-Methoxyethanol, n-Butylacetat und 1-Ethoxy-2-propanol.

**Claims**

1. Process for purifying 1,1,1,2,2-pentafluoropropane (245cb) from a first composition comprising 1,1,1,2,2-pentafluoropropane and trans-1,3,3,3-tetrafluoro-1-propene (1234ze-E) and optionally at least one compound chosen from the group consisting of 1,1-difluoroethane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z) and 3,3,3-trifluoropropene (1243zf), said process comprising the steps of:

a) placing said first composition in contact with at least one organic extracting agent to form a second composition;
b) extractive distillation of said second composition to form:

i) a third composition comprising said organic extracting agent and trans-1,3,3,3-tetrafluoro-1-propene (1234ze-E) and optionally at least one compound chosen from the group consisting of 1,1-difluoroethane (152a), 1,1,1,2-tetrafluoroethane (134a), cis-1,3,3,3-tetrafluoro-1-propene (1234ze-Z), trans-1,2, 3, 3, 3-pentafluoropropene (1225ye-E), cis-1,2,3,3,3-pentafluoropropene (1225ye-Z) and 3,3,3-trifluoropropene (1243zf); and
ii) a stream comprising 1,1,1,2,2-pentafluoropropane;

c) recovery and separation of said third composition to form a stream comprising said organic extracting agent and a stream comprising trans-1,3,3,3-tetrafluoro-1-propene (1234ze-E), and optionally said at least one of the compounds chosen from the group consisting of 1,1-difluoroethane (152a), 1,1,1,2-tetrafluoroethane (134a), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z) and 3,3,3-trifluoropropene (1243zf); preferably, the organic extracting agent separated out in step c) is recycled into step a); and **characterized in that** said organic extracting agent is chosen from the group consisting of ethylamine, bromofluoromethane, 1-bromo-1,2-difluoroethylene, acetaldehyde, 1,1,1-trifluoro-2-propanone, 1,1,1-trifluoro-2-bromoethane, 2,2,2-trifluoroethyl methyl ether, isopropylamine, methyl formate, 2-methoxy-1-propene, diethyl ether, 1,2-epoxypropane, ethanethiol, ethoxyethene, ethylmethylamine, dimethyl sulfide, 2-chloropropane, bromoethane, dimethoxymethane, iodomethane, 2-amino-2-methylpropane, methyl cyclopropyl ether, n-propylamine, isopropylmethylamine, ethanedial, 2-chloro-2-methylpropane, 2-propanethiol, 2-ethoxypropane, methyl t-butyl ether, diethylamine, propanone, methyl acetate, 4-methoxy-2-methyl-2-butanethiol, 2-bromopropane, chloromethoxymethane, 2-butanamine, n-methylpropylamine, tert-butylthiol, isobutanal, methanol, tetrahydrofuran, 1-propanethiol, chlorobromomethane, isopropyl formate, diisopropyl ether, 3-bromopropene, 1-bromopropane, methyl glyoxal, iodoethane, 2-ethoxy-2-methylpropane, 2-bromo-2-methylpropane, 2,2,2-trifluoroethanol, 1-chloro-3-fluoropropane, 1,1,1-trifluoro-2-propanol, 1-butylamine, ethyl acetate, ethanol, butanone, n-propyl formate, 2-ethoxybutane, 2-propanol, acetonitrile, tert-butanol, 1-methoxy-2-methylbutane, 2,2-dimethoxypropane, 2-chloro-2-methylbutane, 1,2-dichloroethane, 1-ethoxy-2-methylpropane, diisopropylamine, 2-butanethiol, 1,2-dimethoxyethane, 3-methyl-2-butanamine, diethoxymethane, 2-methyl-1-propanethiol, isopropyl acetate, 2-iodopropane, di-n-propyl ether, 3-pentylamine, n-methylbutylamine, 2-bromobutane, diethyl sulfide, 1-ethoxybutane, 1-methoxy-2-propanamine, 2-methylbutanal, 2-methoxyethanamine, 2,2-difluoroethanol, 1,2-dichloropropane, propanol, tert-butyl acetate, propionitrile, trichloroacetaldehyde, 2-allyloxyethanol, butanethiol, 1-methoxypentane, ethyl propionate, 2-butanol, 1,2-dimethoxypropane, isopropyl isobutyl ether, 1-chloro-4-fluorobutane, 1-bromo-3-fluoropropane, dioxane, 1-bromobutane, 3-pentanone, 1,1-diethoxymethane, 2-pentanone, 2-methyl-2-butanol, 1-iodopropane, 2-methoxy-1-propanamine, trimethoxymethane, cis-1,3-dichloropropene, n-pentylamine, 3,3-dimethyl-2-butanone, 1,3-dioxane, piperidine, 1-bromo-2-chloroethane, isobutanol, 2-bromo-2-methylbutane, dipropylamine, 2,2,3,3-tetrafluoro-1-propanol, 2-ethoxyethanamine, triethylfluorosilane, sec-butyl acetate, trans-1,3-dichloropropene, 2,2-dimethyl-1-propanol, n-methyl-1,2-ethanediamine, 2,2-diethoxypropane, 1,3,5-trioxane, pyridine, n-methylmorpholine, 3-pentanol, 4-methyl-2-pentanone, 1,2-diaminoethane, isobutyl tert-butyl ether, 2-bromopentane, butyronitrile, 1-butanol, 2,3-dichlorobutane, sec-butyl tert-butyl ether, 1-methoxy-2-propanol, 1,2-propanediamine, 2,6-dimethyl-5-heptenal, 1-bromo-3-methylbutane, 1,3-dichloro-trans-2-butene, 1,3-dichloropropane, 1-(dimethyl-amino)-2-propanol, tetrahydrothiophene, 3-methyl-3-pentanol, 1,2-dibromo-1-fluoroethane, 1,1-diethoxypropane, 1,2,2-trichloropropane, 1-chloro-2-methyl-2-propanol, 2-methoxyethanol, 4,4,4-trifluorobutanol, 2-ethylbutylamine, diethyl carbonate, n-butyl acetate, 1-pentanethiol, 2-chloro-1,1-dimethoxyethane, 2-hexanone, n-ethylethylene-diamine, 3-fluoropropanol, 5-hexen-2-one, 2,3-dichloro-2-methylbutane, 1,1-diethoxy-n,n-dimethylmethanamine, 2-methylpyridine, 1-bromopentane, 2-methoxy-1-propanol, 1,2-dichloro-2-butene, 1-iodobutane, hexanal, 1-ethoxy-2-propanol, 1,2-dibromoethane, 4-methyl-2-pentanol, bromoacetic acid methyl ester, 1,1,2-trichloropropane, 1,2-octanediol, 4-methyl-2-hexanamine, hexylamine, 2-chloro-1-propanol, methoxycyclohexane, 2-(dimethylamino)ethanol, 1,3-dichlorobutane, cyclohexylamine, n-ethyl-2-dimethylaminoethylamine, ethoxyethanol, 3-hexanol, 2-hexanol, 2-methylpyrazine, 2-ethoxy-1-propanol, 1-pentanol, n-ethylmorpholine, 1-methylpiperazine, 1,3-propanediamine, di-n-butyl ether, valeronitrile, (methyleneamino)-acetonitrile, 1,2-dibromopropane, 1,2,3-trichloropropene, 2-heptanamine, 2,3-dimethylbutanol, 1-ethoxyhexane, 1-chloro-3-bromopropane, n,n-diethylethylenediamine, 3-furfural, 2,6-dimethylpyridine, 4-methyl-2-hexanone, 1,1,1-triethoxyethane, 1-methoxy-2-acetoxypropane, 4-methylpyridine, n,n'-diethyl-1,2-ethanediamine, 2,6-dimethyl-morpholine, 2-ethyl-1-butanol, 2-methyl-1-pentanol, methyl hexanoate, 2-propoxyethanol, 1-propoxy-2-propanol and dimethylethanolamine.

2. Process according to Claim 1, **characterized in that** the first composition is an azeotropic or quasi-azeotropic composition comprising 1,1,1,2,2-pentafluoropropane (245cb) and trans-1,3,3,3-tetrafluoro-1-propene (1234ze-E) and obtained at a temperature of from -30°C to 50°C and at a pressure of from 0.5 to 10 bar.

3. Process according to either of the preceding claims, **characterized in that** said organic extracting agent is chosen from the group consisting of ethylamine, isopropylamine, n-propylamine, diethylamine, tetrahydrofuran, ethyl acetate, butanone, 3-pentylamine, 2-methoxyethanamine, dioxane, 2-pentanone, n-pentylamine, 1,3-dioxane, 1,2-diaminoethane, 1,2-propanediamine, 2-methoxyethanol, n-butyl acetate and 1-ethoxy-2-propanol.

4. Process according to any one of the preceding claims, **characterized in that** said stream comprising 1,1,1,2,2-pentafluoropropane formed in step b) is recovered to be used in a process for producing 2,3,3,3-tetrafluoropropene.

5. Process for producing 2,3,3,3-tetrafluoro-1-propene, comprising the steps of:

A) fluorination in the presence of a catalyst for a compound of formula (I) $CX(Y)_2\text{-}CX(Y)_m\text{-}CH_mXY$ in which X and Y independently represent a hydrogen, fluorine or chlorine atom and m = 0 or 1 and/or fluorination in the presence of a catalyst for a compound of formula $(CX_nY_{3-n})CH_pX_{1-p}CH_mX_{2-m}$ (II) in which X is, independently of each other, Cl, F, I or Br; Y is, independently of each other, H, Cl, F, I or Br; n is 1, 2 or 3; and m is 0, 1 or 2; and p is 0 or 1;
B) recovery of a stream comprising 2,3,3,3-tetrafluoro-1-propene, 1,1,1,2,2-pentafluoropropane and at least one compound chosen from the group consisting of 1,1-difluoroethane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z) and 3,3,3-trifluoropropene (1243zf);
C) distillation of the stream recovered in step B) and recovery, at the top of the distillation column, of a stream comprising 2,3,3,3-tetrafluoro-1-propene, and, at the bottom of the distillation column, a stream comprising 1,1,1,2,2-pentafluoropropane and at least one compound chosen from the group consisting of 1,1-difluoroethane (152a), 1,1,1,2-tetrafluoroethane (134a), trans-1,3,3,3-tetrafluoro-1-propene (1234ze-E), cis-1,3,3,3-tetrafluoro-1-propene (1234ze-Z), trans-1,2,3,3,3-pentafluoropropene (1225ye-E), cis-1,2,3,3,3-pentafluoropropene (1225ye-Z) and 3,3,3-trifluoropropene (1243zf);
D) implementation of the process for purifying 1,1,1,2,2-pentafluoropropane as claimed in any one of Claims 1 to 4 using the stream recovered at the bottom of the distillation column in step C); and
E) recycling into step A) of the stream comprising 1,1,1,2,2-pentafluoropropane formed and recovered in step b) of the purification process performed in step D) or dehydrofluorination of the stream comprising 1,1,1,2,2-pentafluoropropane formed in step b) of the process for purifying 1,1,1,2,2-pentafluoropropane performed in step D) in the absence or presence of hydrofluoric acid.

6. Composition comprising 1,1,1,2,2-pentafluoropropane and trans-1,3,3,3-tetrafluoro-1-propene and an organic extracting agent chosen from the group consisting of ethylamine, isopropylamine, n-propylamine, diethylamine, tetrahydrofuran, ethyl acetate, butanone, 3-pentylamine, 2-methoxyethanamine, dioxane, 2-pentanone, n-pentylamine, 1,3-dioxane, 1,2-diaminoethane, 1,2-propanediamine, 2-methoxyethanol, n-butyl acetate and 1-ethoxy-2-propanol.

FIG. 1a

FIG. 1b

FIG. 1c

FIG. 2

**EP 3 394 018 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0864554 A
- WO 03068716 A
- WO 9819982 A
- WO 2010123154 A
- WO 2007079431 A

- EP 939071 A
- WO 2008054781 A
- WO 2008040969 A
- US 4902838 A
- WO 2009118628 A